# EUROPEAN PATENT APPLICATION

(11) **EP 3 747 422 A1**
(43) Date of publication of application: **09.12.2020**
(21) Application number: 19747085.9
(22) Date of filing: 10.01.2019
(51) Int. Cl.: A61K 6/083, A61K 9/19, A61K 31/165, A61K 31/14, B82Y 5/00

(54) **METHOD FOR OBTAINING FUNCTIONALISED POLYMER PARTICLES**

(30) Priority: 02.02.2018 ES 201830096
(71) Applicant: Nanomateriales y Polímeros, S.L., 18016 Armilla (Granada) (ES); Universidad de Granada, E-18071 Granada (ES)
(72) Inventor: TOLEDANO PÉREZ, Manuel, 18071 Granada (ES); OSORIO RUIZ, Raquel, 18071 Granada (ES); MEDINA CASTILLO, Antonio Luis, 18016 Armilla (Granada) (ES)
(74) Representative: Pons
(86) International application number: PCT/ES2019/070011
(87) International publication number: WO 2019/149975

(57) **Abstract**

The present invention relates to a precipitation polymerization method for obtaining spherical polymer particles of a copolymer with statistical topology and chemical structure (acid monomer selected from acrylic or methacrylic)-co-(cross-linker selected from acrylic or methacrylic)-co-(hydroxylated monomer selected from acrylic or methacrylic) and which are functionalised with a divalent cation selected from Zn⁺², Ca+², Mg⁺² and Sr⁺² and/or an antibacterial agent. The invention also relates to said polymeric particles. The invention further relates to a composition comprising the polymeric particles and to the use of the particles to produce a drug.

## Description

The present invention relates to a method of precipitation polymerization to obtain polymeric spherical particles of copolymer with a statistical topology and chemical structure (acid monomer selected from acrylic or methacrylic)-co-(cross-linking agent selected from acrylic or methacrylic)-co-(hydroxylated monomer selected from acrylic or methacrylic) functionalized with a divalent cation selected from Zn⁺², Ca+², Mg⁺² and Sr⁺² and/or an antibacterial agent and relates to said polymeric particles. In addition, the present invention relates to a composition comprising said polymeric particles and to the use of said particles for manufacturing a medicament.

### BACKGROUND OF THE INVENTION

One of the most significant problems which can arise following an endodontic intervention is the appearance of prolonged infections which require another subsequent intervention or which may result in the loss of the dental piece. The main reason for this problem is the imperfect disinfection of the root dentin during the endodontic surgery which results in the existing bacteria not being completely eliminated.

Currently, one of the greatest challenges in endodontic treatments is to completely eliminate the bacteria in the root canal system. The microorganisms that can survive treatment affect periapical healing and facilitate the development of apical lesions. The reactivation of these bacteria has direct and indirect harmful effects through the activation of the metalloproteases. Therefore, an agent is required which can be applied to the walls of the dentin, in the root canal, which has a bactericide effect long term and an inhibitory activity of the metalloproteases.

The search for a biomaterial with a significant antibacterial effect inside the root canals is not a new challenge; various materials have been proposed before:
A triple antibiotic mixture (TAM) has recently been used as an intracanal medicament against the pathogens commonly found in the canal system of the root. This mixture contains ciprofloxacin, metronidazole and minocycline and can effectively eliminate bacteria, but has two significant disadvantages:
- It causes a darkening of the dental structure [Shakouie S, Shahi S, Samiei M, Milani AS, Reyhani MF, Paksefat S, Eskandarinekhad M, Ghasemi N. Effects of different intra canal medicaments on the push out bond strength of endodontic sealers. J Clin Exp Dent. 2017;9(3):e443-e447].
- It changes the bonding strength of the canal sealers [Shokraneh A, Farhad AR, Farhadi N, Saatchi M, Hasheminia SM. Antibacterial effect of triantibiotic mixture versus calcium hydroxide in combination with active agents against Enterococcus faecalis biofilm. Dent Mater J 2014;33(6):733-738.].

Calcium hydroxide Ca(OH)₂ is widely used in post-endodontic treatments. It has been shown that the short and long-term use of Ca(OH)₂ in the root canal could cause negative effects on the chemical and mechanical properties of the dentin of the root, possibly being due to its increased pH which could result in prejudicial effects on the collagen of the surface of the dentin. It has also been shown that the Ca(OH)₂ does not have a significant effect on the resistant microorganisms inside the root canal in the long term.

In addition, the teeth have an internal layer of dentin and a hard external layer of enamel. In humans, the enamel varies in thickness on the surface of the tooth, sometimes being thicker in the upper area, up to 2.5 mm and thinner at the edge of the cement-enamel bond. However, demineralization processes frequently occur in the enamel, which degrade it. This destruction of the enamel results in the exposure of the dentin to the oral environment and sometimes, it is associated with resulting dentin hypersensitivity during the movement of fluids within the dentinal tubules. The commonly used desensitizing agents contain, for example, potassium oxalate providing a temporary occlusion of the tubule by means of oxalate crystals. However, it would be necessary to achieve remineralization of the damaged dentin and a tubular occlusion by means of depositing mineral compounds similar to those lost.

### DESCRIPTION OF THE INVENTION

The present invention relates to a method of precipitation polymerization to obtain polymeric spherical particles of copolymer with a statistical topology and chemical structure (acid monomer selected from acrylic or methacrylic)-co-(cross-linking agent selected from acrylic or methacrylic)-co-(hydroxylated monomer selected from acrylic or methacrylic) functionalized with a divalent cation selected from Zn⁺², Ca⁺², Mg⁺² and Sr⁺² and/or an antibacterial agent comprising a first step of preparing a monomeric solution with the monomers of the copolymer, a cross-linking agent, a solvent, an initiator and particles with fractal surface and a subsequent step of incubating the polymeric particles in an aqueous solution comprising Zn⁺², Ca⁺², Mg⁺², Sr⁺² and/or an antibacterial agent whose aim is to functionalize the polymeric particles.

A copolymer is a macromolecule composed of two or more monomers or different repetitive units which can be joined in different manners by means of chemical bonds. In the present invention, the term "copolymer with statistical topology" refers to statistical copolymers, that is to say, a copolymer in which the distribution of the monomers in the chain is random since all the monomers present in the solution have the same affinity/probability to react both with monomers of the same chemical nature (with themselves) and with monomers of a different chemical nature.

When the functionalized polymeric particles have dimensions less than 300 nm, they can occupy the dentin tubules, such that said particles can be used tomanufacture a medicament with the capacity to remineralize the dentin and/or for the treatment and/or prevention of bacterial infections of the dentin of the root canal. Therefore, said particles can be used to manufacture a medicament for the treatment and/or prevention of the hypersensitivity of the dentin and for manufacturing a medicament for the treatment and/or prevention of infectious recurrences following an endodontic intervention.

In a first aspect, the present invention relates to a method of obtaining polymeric particles by precipitation polymerization (hereinafter referred to as "method of the present invention"),
- comprising a copolymer with statistical topology and a chemical structure (acid monomer selected from acrylic or methacrylic)-co-(cross-linking agent selected from acrylic or methacrylic)-co-(hydroxylated monomer selected from acrylic or methacrylic)
- having particle dimensions of less than 1000 nm
- being functionalized with a divalent cation selected from Zn⁺², Ca⁺², Mg⁺² and Sr⁺², an antibacterial agent and/or any of the combinations thereof,
characterized by being a precipitation polymerization comprising the following steps
a) preparing a solution comprising:
   - an acid monomer selected from acrylic or methacrylic in a proportion of 0.05% and 10% by weight with respect to the total weight of the solution
   - a cross-linking agent selected from acrylic or methacrylic in a proportion of 0.05% and 10% by weight with respect to the total weight of the solution
   - a hydroxylated monomer selected from acrylic or methacrylic in a proportion of 0.05% and 10% by weight with respect to the total weight of the solution
   - a solvent comprising at least acetonitrile, acrylonitrile, propionitrile, benzonitrile, butyronitrile, methyl ethyl ketone (butanone), ethyl acetate, 1,2-dimethoxyethane or a combination thereof, in a proportion between 80% and 99.8% by weight with respect to the total weight of the solution
   - an initiator in a proportion of 0.01% and 2% by weight with respect to the total weight of the solution
   - particles with fractal surface in a proportion of 0.009% and 2% by weight with respect to the total weight of the solution
b) eliminating the oxygen present in the solution obtained in step (a);
c) heating the solution obtained in (b) to a temperature between 30°C and the boiling point of the solvent used in step (a);
d) isolating the polymeric particles obtained in step (c), rinsing and drying;
e) functionalizing the polymeric particles obtained in step (d) by means of incubating them in an aqueous solution comprising Zn⁺², Ca⁺², Mg⁺², Sr⁺² and/or an antibacterial agent, respectively, to obtain a suspension, wherein the percentage by weight of the polymeric particles in the suspension is between 0.01% and 30%; and
f) isolating the polymeric particles functionalized in step (e) and drying.

In a preferred embodiment of the method of the invention, the polymeric particles obtained have particle dimensions of less than 300 nm.

In another preferred embodiment of the present invention, the polymeric particles obtained are spherical.

In another preferred embodiment of the method of the present invention, the acid monomer of step (a) is selected from methacrylic acid (MA), acrylic acid (AA), 2-carboxyethyl acrylate, mono-2-(methacryloyloxy)ethyl maleate, 2-carboxyethyl acrylate oligomers, 2-bromoacrylic acid, 2-bromomethacrylic acid, 2-ethylacrylic acid, 2-propylacrylic acid and 2-trifluoromethyl acrylic acid.

In another preferred embodiment of the method of the present invention, the cross-linking agent of step (a) is selected from ethylene glycol dimethacrylate (EDMA), 3-(acryloyloxy)-2-hydroxypropyl methacrylate, bis[2-(methacryloyloxy)ethyl] phosphate, bisphenol A propoxylate diacrylate, 1,3-butanediol diacrylate, 1,4 butanediol diacrylate, 1,3-butanediol dimethacrylate, di(trimethylpropane) tetracrylate, diurethane dimethacrylate, glycerol 1,3-diglycerolate diacrylate, glycerol dimethacrylate, glycerol propoxylate (1PO/OH) triacrylate, 1,6-hexanediol diacrylate, neopentyl glycol diacrylate, pentaerythritol diacrylate monostearate, pentaerythritol tetra-acrylate and trimethylolpropane propoxylate triacrylate.

In another preferred embodiment of the method of the present invention, the hydroxylated monomer of step (a) is selected from 2-hydroxyethyl methacrylate (HEMA), 2-hydroxyethyl acrylate (HEA), hydroxypropyl acrylate, hydroxypropyl methacrylate, 4-hydroxybutyl acrylate, hydroxybutyl methacrylate and 2-hydroxy-3-phenoxypropyl acrylate.

In another preferred embodiment of the method of the present invention, the initiator of step (a) is selected from 2,2'-azobis(2-methylpropionnitrile) (AIBN), 1,1'azobis(cyclohexanecarbonitrile) (ACHN), 2,2'-azobis (2-methylpropionamidine) 2,2'-dihydrochloride (AAPH), 4,4'-azobis(4-cyanovaleric acid) (ACVA), tert-butyl hydroperoxide, cumene hydroperoxide, 2,5-di(tert-butylperoxide)-2,5-dimethyl-3-hexyne, dicumyl peroxide and 2,5-bis(tert-butylperoxide)-2,5-dimethylhexane.

Step (a) of the method of the invention is carried out in the presence of a solvent comprising at least acetonitrile, acrylonitrile, propionitrile, benzonitrile, butyronitrile, methyl ethyl ketone (butanone), ethyl acetate, 1,2-dimethoxyethane or a combination thereof.

When step (a) of the method is carried out in the presence of water, the polymeric particles are agglomerated, which is undesirable and should be avoided. Therefore, in a preferred embodiment of the method of the present invention, the solvent used to obtain the solution of step a) is not water.

When step (a) of the method is carried out in pure solvents with a high value of the Hansen solubility parameter to form a hydrogen bridge (*δₕ*) such as alcohols: 1-propanol, 2-propanol, ethanol, methanol, butanol, the polymeric chains of the copolymer are maintained solvated up to high conversion values, undesired amorphous macrogels being obtained.

When step (a) of the method is carried out in pure solvents with a high value of the Hansen solubility parameter to give dispersive strengths (*δ_{d}*) such as xylene, toluene, ethylbenzene, octane, heptane, hexane, undesired, non-spherical particles are obtained of 1500 nm diameter formed by aggregation/coagulation of smaller particles. When step (a) of the method is carried out with
- at least acetonitrile, acrylonitrile, propionitrile, benzonitrile, butyronitrile, methyl ethyl ketone (butanone), ethyl acetate, 1,2-dimethoxyethane or a combination thereof,
- and some of the solvents previously mentioned,
particle dimensions of less than 1000 nm are obtained.

In a preferred embodiment of the method of the present invention, the solvent is pure and is selected from acetonitrile, acrylonitrile, propionitrile, benzonitrile, butyronitrile, methyl ethyl ketone (butanone), ethyl acetate, 1,2-dimethoxyethane and a combination thereof

In the present invention, "particles with fractal surface" are understood as a particle comprising a fractal surface, that is to say, a surface whose geometry comprises a fractal dimension, the fractal dimension being a real number which generalizes the ordinary dimension concept for geometric objects which do not allow tangent space. In a differential geometry, tangent space is the combination associated with each point of a distinguishable variety formed by all the tangent vectors to said point.

Examples of particles with hydrophilic fractal surface are porous silica SiO₂ or alumina Al₂O₃ nanoparticles.

Silica SiO₂ is a porous and hydrophilic material with a high adsorption capacity of polar polymers and copolymers by means of: complexing between the polar groups (COOH and OH) of the monomers comprising the copolymer and the mono and divalent cations present in the silica, by a hydrogen bridge, by electrostatic interactions, etc. The SiO₂ nuclei behave like fractal hydrophilic surfaces capable of lowering the nucleation barrier practically to zero; there where nucleation phenomena occur (metal solidification, liquid condensation, mineral solidification, etc.), causing the nucleation phenomena to preferably take place on the surface of said nuclei, via heterogeneous nucleation. In the precipitation polymerization processes, nucleation phenomena also take place; therefore the SiO₂ nuclei behave in the same manner: the presence of SiO₂ nuclei means that the nucleation mechanism in the precipitation polymerization is exclusively via heterogeneous nucleation.

Step (a) of the method of the invention requires the presence of particles with fractal surface; the precipitation polymerization reaction takes place by means of a heterogeneous nucleation mechanism in the presence of said particles with fractal surface as is shown below. The heterogeneous nucleation in precipitation polymerization consists of the precipitation of the polymeric chains growing on areas where a solid surface already exists, that is to say, on the fractal surface of the particles. In the following diagram, SiO₂ nuclei are used as fractal surface particles.

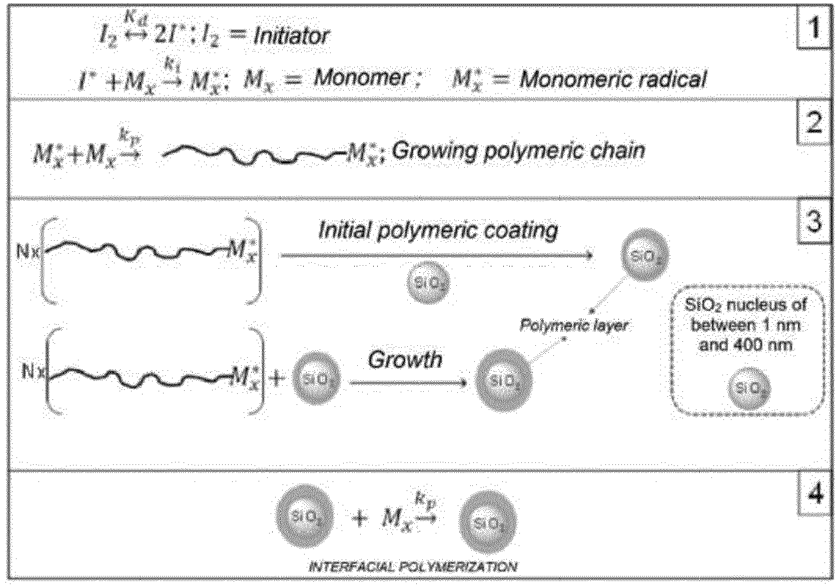

Step 1 of the proposed mechanism consists of the initiation and formation of roots from the acid monomer, the hydroxylated monomer, the cross-linking agent, the solvent and the initiator in the presence of SiO₂ nuclei.

Step 2 is the step of propagation and growth of the polymeric chains in solution.

Step 3 consists of 2 stages:
a) precipitation; the polymeric chains grow solvated until reaching the maximum possible size in solution to subsequently precipitate on the SiO₂ nuclei, forming the first SiO₂ copolymer particles.
b) growth; once all the SiO₂ nuclei are in the SiO₂ copolymer form, the subsequent precipitations cause growth of the initial SiO₂ copolymer particles.

Step 4 corresponds to the interfacial polymerization phenomenon due to the possible adsorption of monomers on the surface of the particles and the subsequent propagation thereof on residual developing chains which can remain on the surface of the particles.

The result of carrying out the reaction of precipitation polymerization in the presence of particles with fractal surface such as for example SiO₂ nuclei is the reduction of the polymeric particle size by up to 4 times below the size obtained under the same experimental conditions in the absence of said particles with fractal surface.

In addition, the polydispersity index of the polymeric particles is also reduced when the precipitation polymerization is carried out in the presence of particles with fractal surface.

In a preferred embodiment of step (a) of the method of the present invention, the particles with fractal surface of step (a) are SiO₂ or Al₂O₃ nuclei with a particle size of between 1 nm and 400 nm. More preferably, the particle size of the SiO₂ or Al₂O₃ nuclei is between 1 nm and 50 nm.

Step (b) of the method of the present invention relates to the elimination of the oxygen present in the solution obtained in step (a). Said step (b) is preferably carried out by means of purging with nitrogen.

Step (c) of the method of the present invention relates to the heating of the solution obtained in (b) to a temperature between 30°C and the boiling point of the solvent used in step (a).

Step (d) of the method of the present invention relates to the isolation of the polymeric particles obtained in step (c), preferably by means of centrifugation and elimination of the supernatant.

Once rinsed and dried, the polymeric particles obtained in step (d) are functionalized in step (e) by means of incubating them in an aqueous solution comprising Zn⁺², Ca⁺², Mg⁺², Sr⁺² and/or an antibacterial agent, respectively, to obtain a suspension, wherein the percentage by weight of the polymeric particles in the suspension is between 0.01% and 30%. Aqueous solutions of ZnCl₂, CaCl₂ are preferred for the functionalization with the cations Zn⁺² and Ca⁺², respectively.

The mechanism by which the polymeric particles are functionalized with divalent cations such as Zn⁺², Ca+², Mg⁺² or Sr⁺² is due to the high affinity constants exhibited by the carboxy (COOH) and hydroxyl (OH) groups present on the surface of the polymeric particles for the formation of complexes with said divalent cations, while the functionalization with the antibacterial agent can be carried out by means of covalent bonding or by means of non-covalent interactions: hydrophobic, hydrogen bridge, dispersive, ionic, etc., depending on the chemical structure of the antibacterial used.

In a preferred embodiment of the method of the present invention, the antibacterial agent of step (e) is selected from the list comprising tetracycline, oxytetracycline, doxycycline hyclate, doxycycline hydrochloride, 4-epi-chlortetracycline hydrochloride, neomycin, gentamycin, tobramycin, sulfates and derivatives thereof, macrolides such as erythromycin, penicillins such as ampicilin and amoxycylin, vancomycin, cephalosporins and non-toxic antiseptics for oral use such as chlorhexidine in any of its forms: chlorhexidine digluconate and chlorhexidine acetate.

Another aspect of the present invention relates to polymeric particles obtained by means of the previously described method (hereinafter referred to as "particles of the invention") where said particles
- comprise a copolymer with statistical topology and a chemical structure (acid monomer selected from acrylic or methacrylic)-co-(cross-linking agent selected from acrylic or methacrylic)-co-(hydroxylated monomer selected from acrylic or methacrylic)
- have particle dimensions of less than 1000 nm,
- and are functionalized with a divalent cation selected from Zn⁺², Ca⁺², Mg⁺² and Sr⁺², an antibacterial agent and/or any of the combinations thereof.

In a preferred embodiment of the particles of the invention, the particle dimensions are less than 300 nm.

Another aspect of the present invention relates to a composition comprising the particles of the present invention whose technical characteristics have been mentioned previously.

Another aspect of the present invention relates to a pharmaceutically-acceptable composition comprising the particles of the invention

The composition of the invention can be presented in a formulation selected from the list consisting of: cream, emulsion, anhydrous composition, aqueous dispersion, oil, milk, balm, foam, lotion, gel, hydroalcoholic solution, hydroglycolic gel, hydrogel, liniment, serum, ointment, mousse, salve, powder, vaporizer, aerosol, capsule, pill, tablet, granulate, solution, suspension, syrup, film, etc.

In a particular embodiment of the present invention, the particles of the invention with dimensions less than 300 nm are capable of
- being used as an antibacterial agent capable of slowly releasing the active ingredient inside the root canals over long periods of time or acting 'per se' from the functionalized polymeric particle
- being used in odontological applications such as the treatment of dentin hypersensitivity, facilitating the remineralization of the dentin since said particles (functionalized with Zn⁺² or Ca⁺²) can occlude the tubules of the dentin.

In turn, the term "treatment" or "to treat" in the context of this document relates to the administration of a compound or a composition according to the invention to improve or eliminate a disease, pathological condition or one or more symptoms associated with said disease or condition in a patient. "Treatment" also encompasses the improvement or elimination of the physiological consequences of the disease.

Specifically, the concept "to treat" can be interpreted as:
i. Preventing the disease or pathologic condition, that is to say, impeding or delaying its appearance;
ii. Inhibiting the disease or pathologic condition, that is to say, stopping the development thereof;
iii. Alleviating the disease or the pathological condition, that is to say, causing the regression of the disease or pathological condition;
iv. Stabilizing the disease or pathological condition.

The term "medicament" as is used in this description, refers to any substance used for the treatment of diseases or pathological conditions in a human or animal.

One advantage of the particles of the invention is the use thereof as an antibacterial agent, in the long-lasting effect thereof and the synergy between the bacterial inhibition and the inhibition of the activity of the metalloproteases. The metalloproteases are powerfully inhibited by the doxycycline and have a chelate effect on the divalent cations. It should be pointed out that the particles of the invention competitively inhibit the collagenolytic action (destruction of tooth and bone) of the metalloproteases, that is to say, they inhibit the dentin metalloproteases and stop the destruction of the collagen.

It has been verified that the biocompatibility of the particles of the invention is greater than 90% when they were analyzed against human fibroblasts, therefore they can be a useful tool in endodontic revascularization techniques.

In addition, the particles of the invention are compatible with all the known materials used for sealing the canal of the root, therefore they can be used in the root canal reducing to a large extent the recurrences and the need for secondary surgical interventions after the endodontic interventions.

An additional application of these particles is the topical administration on the dentin of the root surface or cervical dentin, being capable of closing the dentin tubules, inhibiting the metalloproteases of the dentin and restoring the mechanical properties of the dentin surface due to the remineralizing properties thereof. Therefore, hypersensitivity is reduced by means of the occlusion of tubules and the advance of damage is avoided with the improvement to the mechanical properties of the dentin and inhibition of the metalloproteases (involved in the advance of this dentin erosion process).

Therefore, another aspect of the invention relates to the use of the particles of the invention (hereinafter referred to as "use of the present invention"),
- comprising a copolymer with statistical topology and a chemical structure (acid monomer selected from acrylic or methacrylic)-co-(cross-linking agent selected from acrylic or methacrylic)-co-(hydroxylated monomer selected from acrylic or methacrylic)
- having particle dimensions of less than 1000 nm,
- being functionalized with a divalent cation selected from Zn⁺², Ca⁺², Mg⁺² and Sr⁺², an antibacterial agent and/or any of the combinations thereof,
for the manufacturing of a medicament.

In addition, the present invention relates to the particles of the invention,
- comprising a copolymer with statistical topology and a chemical structure (acid monomer selected from acrylic or methacrylic)-co-(cross-linking agent selected from acrylic or methacrylic)-co-(hydroxylated monomer selected from acrylic or methacrylic)
- having particle dimensions of less than 1000 nm,
- being functionalized with a divalent cation selected from Zn⁺², Ca⁺², Mg⁺² and Sr⁺², an antibacterial agent and/or any of the combinations thereof,
for their use as a medicament.

In a preferred embodiment of the present invention, the use of the particles of the invention relates to the manufacture of a medicament for the treatment and/or prevention of diseases derived from the activity of metalloproteases such as cancer or inflammatory processes.

In turn, the present invention relates to the polymeric particles of the invention,
- comprising a copolymer with statistical topology and a chemical structure (acid monomer selected from acrylic or methacrylic)-co-(cross-linking agent selected from acrylic or methacrylic)-co-(hydroxylated monomer selected from acrylic or methacrylic)
- having particle dimensions of less than 1000 nm,
- being functionalized with at least one divalent cation selected from Zn⁺², Ca⁺², Mg⁺² and Sr⁺²,
for their use as a medicament for the treatment and/or prevention of diseases derived from the activity of metalloproteases such as cancer or inflammatory processes.

In another preferred embodiment of the present invention, the use of the polymeric particles of the present invention relates to the manufacture of a medicament for the remineralization of the dentin where the particles are functionalized with at least one divalent cation selected from Zn⁺², Ca⁺², Mg⁺² and Sr⁺², an antibacterial agent and/or any of the combinations thereof.

In turn, the present invention relates to the polymeric particles of the invention,
- comprising a copolymer with statistical topology and a chemical structure (acid monomer selected from acrylic or methacrylic)-co-(cross-linking agent selected from acrylic or methacrylic)-co-(hydroxylated monomer selected from acrylic or methacrylic)
- having particle dimensions of less than 300 nm,
- being functionalized with at least one divalent cation selected from Zn⁺², Ca⁺², Mg⁺² and Sr⁺²,
for their use as a medicament for the remineralization of the dentin.

In another preferred embodiment of the present invention, the use of the particles of the invention relates to the manufacture of a medicament for the treatment and/or prevention of bacterial infections of the root dentin where the particles are functionalized with at least one antibacterial agent.

In addition, the present invention relates to the polymeric particles,
- comprising a copolymer with statistical topology and a chemical structure (acid monomer selected from acrylic or methacrylic)-co-(cross-linking agent selected from acrylic or methacrylic)-co-(hydroxylated monomer selected from acrylic or methacrylic)
- having particle dimensions of less than 300 nm,
- being functionalized with at least one antibacterial agent,
for their use as a medicament for the treatment and/or prevention of bacterial infections of the root dentin.

In another preferred embodiment of the present invention, the use of the particles of the invention relates to the manufacture of a medicament for the remineralization of the dentin and for the treatment and/or prevention of bacterial infections of the dentin, where the particles are functionalized with at least one divalent cation selected from Zn⁺², Ca⁺², Mg⁺² and Sr⁺² and with at least one antibacterial agent.

The present invention also relates to the particles of the invention,
- comprising a copolymer with statistical topology and a chemical structure (acid monomer selected from acrylic or methacrylic)-co-(cross-linking agent selected from acrylic or methacrylic)-co-(hydroxylated monomer selected from acrylic or methacrylic)
- having particle dimensions of less than 300 nm,
- being functionalized with at least one divalent cation selected from Zn⁺², Ca⁺²_{'} Mg⁺² and Sr⁺² and with at least one antibacterial agent.
for the remineralization of the dentin and for the treatment and/or prevention of bacterial infections of the root dentin.

In another preferred embodiment of the present invention, the use of the present invention relates to the manufacture of a medicament for the treatment and/or prevention of the hypersensitivity of the dentin.

The present invention also relates to the polymeric particles of the invention,
- comprising a copolymer with statistical topology and a chemical structure (acid monomer selected from acrylic or methacrylic)-co-(cross-linking agent selected from acrylic or methacrylic)-co-(hydroxylated monomer selected from acrylic or methacrylic)
- having particle dimensions of less than 300 nm,
- being functionalized with a divalent cation selected from Zn+2, Ca+2, Mg+2 and Sr+2, an antibacterial agent and/or any of the combinations thereof,
for the treatment and/or prevention of the hypersensitivity of the dentin

In another preferred embodiment of the present invention, the use of the particles of the present invention relates to the manufacture of a medicament for the treatment and/or prevention of infectious recurrences after an endodontic intervention.

In turn, the present invention relates to the polymeric particles of the invention,
- comprising a copolymer with statistical topology and a chemical structure (acid monomer selected from acrylic or methacrylic)-co-(cross-linking agent selected from acrylic or methacrylic)-co-(hydroxylated monomer selected from acrylic or methacrylic)
- having particle dimensions of less than 300 nm, and
- being functionalized with a divalent cation selected from Zn⁺², Ca⁺², Mg⁺² and Sr⁺², an antibacterial agent and/or any of the combinations thereof,
for the treatment and/or prevention of infectious recurrences after an endodontic treatment.

Throughout the description and the claims, the word "comprise" and its variant do not intend to exclude other technical characteristics, additions, components or steps. For the person skilled in the art, other objects, advantages and characteristics of the invention will emerge in part from the description and in part from the practice of the invention. The following examples and figures are provided for illustration and do not intend to be limiting to the present invention.

### BRIEF DESCRIPTION OF THE FIGURES

Fig. 1. Scanning electron microscope (SEM) images of the particles obtained by precipitation polymerization in glass vials (A) and in Nalgene® (plastic) bottles (B).
Fig. 2. Optical microscope image of the particles obtained in glass vials.
Fig. 3. Comparison of particle sizes and the polydispersity index PDI for the polymeric particles obtained by means of precipitation polymerization carried out in Nalgene® plastic bottles and in glass vials.
Fig. 4. Comparison of particle sizes and the polydispersity index (PDI) for the particles obtained by means of precipitation polymerization carried out in Nalgene® plastic bottles in presence of increasing quantities of glass.
Fig. 5. Optical microscope images of the particles obtained in the presence of the inorganic nanoparticles NTI4 (A) and NTI5 (B).
Fig. 6. Size of the polymeric particles and the polydispersity index (PDI) values vs. concentration of inorganic nanoparticles NTI4 present in the polymerization medium.
Fig. 7. Optical microscope images of the polymeric particles obtained by precipitation polymerization in the presence of the inorganic nanoparticles NTI4-F (A), NTI4-G (B) and NTI4-H (C).
Fig. 8. Scanning electron microscope images of the polymeric particles of 1500 nm obtained in toluene (A), of 250 nm obtained in acetonitrile (B), of 600 nm obtained in a mixture of acetonitrile/2-propanol/toluene using the following fractions in volume of each solvent Φ_{Toluene}=0.73 Φ_{Acetonitrile}=0.25 Φ₂₋ₚᵣₒₚₐₙₒₗ= 0.02 (C) of 1200 nₘ obtained in a mixture of acetonitrile/2-propanol/toluene using the following fractions in volume Φ_{Toluene}=0.035 Φ_{Acetonitrile}=0.90 Φ₂₋ₚᵣₒₚₐₙₒₗ=0.015 (D) and macrogels obtained in 2-propanol (E).
Fig. 9. (A) Average values and standard deviation (SD) of doxycycline release (µg/mL per mg of Dox particles) at each time interval. (B) Doxycycline release (%) per mg of Dox particles at each time interval.
Fig. 10. Survival of *P.gingivalis* (number of viable cells) after incubation with the different particles at different time intervals. The survival value after incubation in phosphate buffer is used as the reference control in all cases.

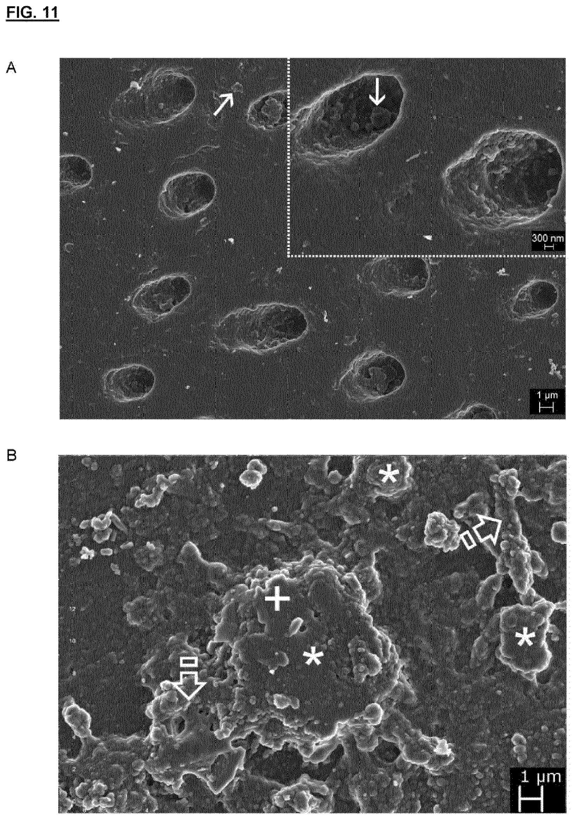

Fig. 11 A) Scanning electron microscope (FESEM) image of the untreated cervical dentin, after 7 days of storage. After this period, some small crystals or mineral deposits (indicated with arrows) were found in the intertubular and peritubular dentin (ID and PD respectively). All the rest of the surface appears free of mineral deposits. B) Scanning electron microscopy (FESEM) image of the cervical dentin treated with Zn particles after 7 days of storage. The surface of the dentin exhibited clustered non-crystalline mineral deposits as a dense network of calcium phosphates with rounded (asterisks) or amorphous (arrows) shapes with no observation of the tubules. The spectra obtained of these deposits by way of the analysis of dispersive energy shows an elemental composition of phosphorous (P) and calcium (Ca).
Fig. 12. Percentage of reduction to the dentin fluid flow (DFF) as a function of the samples analyzed at 24 hours and 7 days.
Fig. 13. A) Average values and standard deviation of nanohardness (Hi) (GPa) on the cervical dentin surfaces both in the peritubular and intertubular area after the application of the different functionalized polymeric particles to the experimental groups at 24 hours and 7 days. B) Young's elasticity modulus (Ei) (GPa) on the cervical dentin surfaces both in the peritubular and intertubular area after the application of different functionalized polymeric particles to the experimental groups at 24 hours and 7 days.

### EXAMPLES

The invention will be illustrated below by means of tests carried out by the inventors which reveal the effectiveness of the product of the invention.

### Example 1: Synthesis of polymeric particles of functionalized MA-co-EDMA-co-HEMA

Spherical polymeric particles of MA-co-EDMA-co-HEMA were prepared by means of precipitation polymerization with thermal initiation using the following monomeric system: 2-hydroxyethyl methacrylate (HEMA), methacrylic acid (MA) and the cross-linking agent ethylene glycol dimethacrylate (EDMA); in acetonitrile (ANC), using azobisisobutyronitrile (AIBN) as the thermal initiator.

The composition of the supply for the precipitation polymerization reaction as well as the experimental conditions used in the reaction are shown in the following Table 1.

**Table 1. Starting composition and experimental conditions**

| | |
|---|---|
| [EDMA] mol/L | 0.063 |
| [MAA] mol/L | 0.081 |
| [HEMA] mol/L | 0.036 |
| [AIBN] mg/mL | 0.65 |
| T _{reaction} (°C) | 82 |
| % monomers vs solvent (m/m) | 4 |

Before carrying out the polymerizations, the monomers were purified and once the solutions with the monomers and the initiator were prepared, the systems were cooled and purged with N₂ for 5 mins with the aim of eliminating the O₂ present in the medium. The reactions were carried out at 82°C in an oil bath with temperature control. Once the polymerizations had finished, the particles were centrifuged, the supernatant was eliminated by decantation, they were rinsed 3 times with 30 ml of methanol and they were dried in a vacuum oven at 70°C to constant weight; once dried, suspensions thereof were prepared in MeOH and the particle sizes were measured by means of the dynamic light scattering technique (DLS) using a Zetasizer Nano ZS90 device from the company Malvern.

When the precipitation polymerization is carried out in vials of different composition, different diameters are surprisingly obtained for the spherical particles of MA-co-EDMA-co-HEMA.

A first experiment consisted of carrying out the invention in a 20 ml glass vial and in a 100 ml Nalgene® plastic bottle.

The Nalgene® plastic brand is a highly inert plastic resistant to chemical and physical agents designed for carrying out chemical reactions at high pressure and temperature.

In order to corroborate the difference in the particle size obtained as a function of the recipient used (glass vial or Nalgene® plastic bottle), the syntheses were repeated 5 times in each one of the recipients, the following results being obtained
- an average particle size of 210 ± 25 nm for the reactions carried out in glass with a polydispersity index (PDI) value of 0.004 ± 0.001 and a yield (R) of 65 ± 9%. Note that the yields were calculated as the percentage by weight of the particles obtained with respect to the weight of monomers used in the supply.
- For the synthesis carried out in Nalgene® plastic bottles, particles sizes of 880 ± 50 were obtained with a PDI of 0.1 ± 0.035 and a yield of 67± 8%

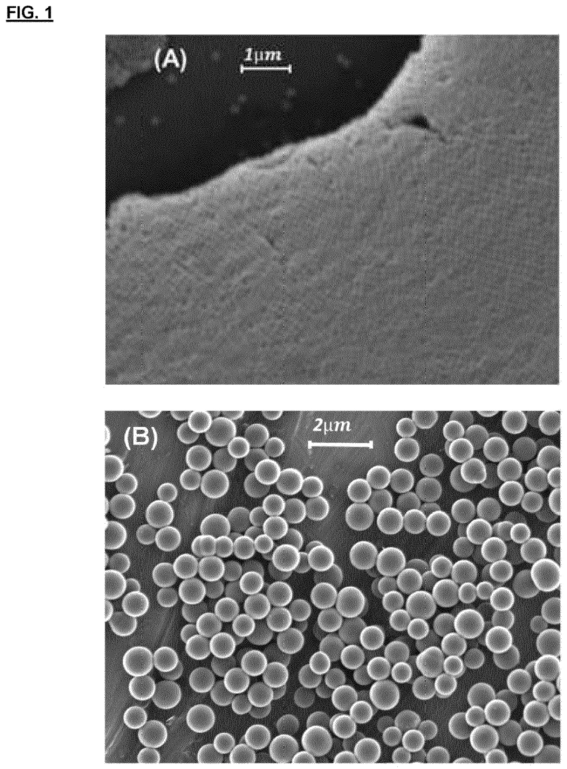

Fig. 1 shows scanning electron microscope (SEM) images of the spherical polymeric particles obtained by precipitation polymerization in glass vials (A) and in Nalgene® (plastic) bottles (B).

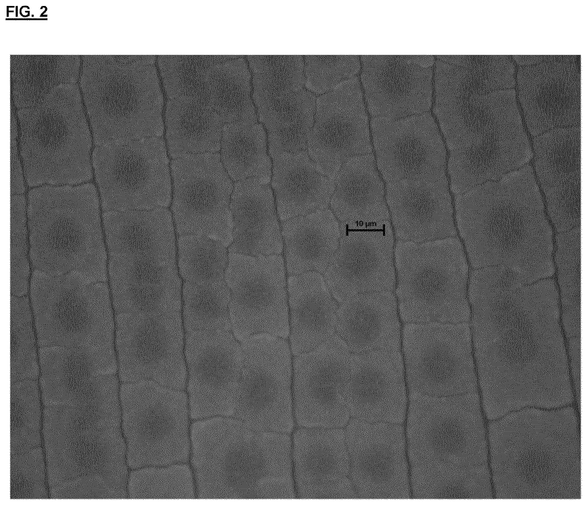

Fig. 2 shows an optical microscope image of highly monodisperse spherical polymeric particles obtained in glass vial and shown in Fig. 1. In Fig. 2 of the optical microscopy, it can be observed how the polymeric particles, when dried, produce a cracked surface formed by macrostructures of approximately 10x30 µm in the form of a compact bundling and increased monodispersity.

The following experiment consisted of the study of the particle size and of the polydispersity index (PDI) as a function of the reaction time in both recipients (20 ml glass vials and 100 ml Nalgene® plastic bottles). The reaction times selected were: 5, 10, 20, 30, 60, 120, 210 and 360 mins. The cloud times, that is to say, the time required to visually observe turbidity in the system due to the formation of the first nuclei in suspension, occur in both cases at approximately 10 mins of reaction. The reactions were stopped by rapid cooling and exposure to oxygen; the particle sizes and polydispersity index values were subsequently measured by DLS.

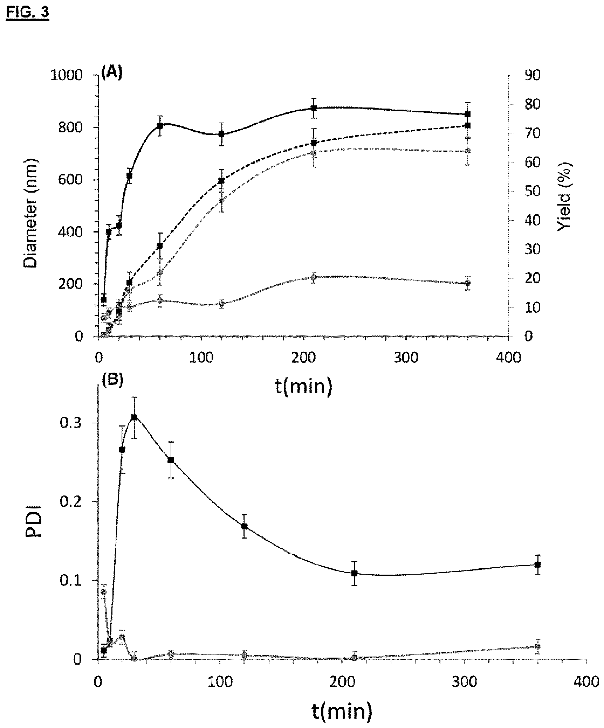

In Fig. 3, the results obtained in this experiment are represented.

In Fig. 3, the comparison between the particles sizes obtained carrying out the polymerizations in 100 ml Nalgene® plastic bottles: black lines: size (-■-) and yield (••••■••••), and in 20 ml glass vials: gray lines: size (-•-) and yield (···•···) (A), comparison between the values of the polydispersity index PDI for the polymerizations carried out in Nalgene® bottles: black line (-■-) and in glass; gray line (-•-) (B) is shown.

In Fig. 3A, it can be observed how the yields are the same in both systems (glass and plastic) and how the polymeric particles synthesized in glass are smaller and extremely monodisperse. It can be concluded that the reduction of the particle size is not produced by an inhibition of the polymerization at early stages due to the system used.

In order to corroborate that the lixiviation of any component of the glass produces the reduction of the particle size, three replicas of the polymerization were carried out in Nalgene® plastic bottles to which broken and milled glasses at a size of 3-5 mm coming from glass vials had been added. Once the polymerization was finished, the glass was removed, the particles were rinsed three times with MeOH and the size thereof was measured by DLS, an average value of 190 ± 15 nm being obtained, with a polydispersity index of 0.0026 ± 0.0008 and a yield of 65 ± 6% being obtained. In view of these results, we can confirm that it is the lixiviation of any component of the glass which produces a reduction of the particle size and the polydispersity index in the precipitation polymerization reaction.

The predominant composition of the glasses is silica SiO₂ in a proportion between 70% and 95%. Pure silica melts at a very high temperature, therefore, the glasses with a high content of SiO₂ are difficult to process and are expensive; for this reason, different additives are used to lower the melting temperature and reduce the cost of the production process which, in turn, modifies the mechanical properties of the glasses, making them more fluid, more fragile, brittle, porous, etc. The following additives stand out: CaO, Na₂O, K₂O, Al₂O₃, MgO, etc. It is in the cooling processes of the glasses where nucleation phenomena are produced capable of producing nanometric SiO₂ nuclei which are trapped in the structure of the glass and which therefore can lixiviate when the glasses are exposed to different liquid mediums.

The following experiment intended to determine the minimum quantity of glass required to obtain particles with diameters less than 300 nm. To this end, polymerizations by precipitation reactions were carried out like those mentioned previously in Nalgene® plastic bottles which contained different quantities of glass coming from 20 ml glass vials, in the reaction medium. Table 2 shows the results of this experiment.

**Table 2. Particle size vs. quantity of glass added in the reaction.**

| Experiment | Glass mass (g) | Polymeric particle size (nm) | Polydispersity index PDI | Yield (%) |
|---|---|---|---|---|
| 1 | 26 | 212 ± 10 | 0.006 ± 0.004 | 69 ± 7 |
| 2 | 20 | 243 ± 15 | 0.002 ± 0.008 | 67 ± 10 |
| 3 | 15 | 255 ± 18 | 0.034 ± 0.022 | 67 ± 10 |
| 4 | 10 | 301 ± 9 | 0.044 ± 0.009 | 67 ± 10 |
| 5 | 5 | 441 ± 12 | 0.009 ± 0.005 | 64 ± 9 |
| 6 | 2 | 665 ± 25 | 0.067 ± 0.006 | 65 ± 10 |

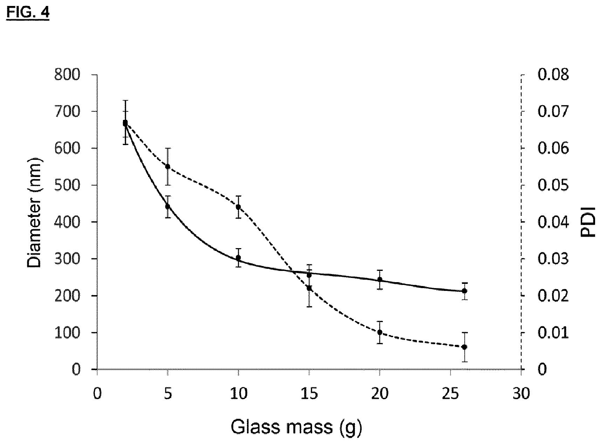

Fig. 4 shows the particle size and the polydispersity index (PDI) for the polymeric particles obtained by means of precipitation polymerization carried out in Nalgene® plastic bottles in presence of growing quantities of glass.

In Fig. 4, it can be observed how 15 g of glass is sufficient for the particle size to be below 300 nm. In addition, in said Fig. 4, it can be observed how, for all cases, the values of the polydispersity index are extremely low, indicating a very high monodispersity of the particles obtained. The particles obtained in this experiment with sizes below 300 nm exhibited similar structures to those of Fig. 2 under the optical microscope.

With the aim of identifying whether the component of the glass which lixiviates and produces the reduction of the particle size and of the polydispersity index PDI in precipitation polymerization, are the nanometric SiO₂ nuclei, the precipitation polymerization was carried out in Nalgene® plastic vials in the same experimental conditions as in the previous cases and in the presence of a fixed quantity of different types of inorganic nanoparticles based on TiO₂ of which only one of them is covered with SiO₂.

The inorganic nanoparticles used in this experiment are detailed below:
- NTI-1: Anatase titanium dioxide (TiO2), purity: 99.5%, particle size: 20 nm, supplied by io-li-tec nanomaterials.
- NTI-2: Rutile titanium dioxide (TiO2), particle size: 10 nm distributed by Dupont.
- NTI-3: Rutile titanium dioxide (TiO2), purity 99.5%, particle size 10-30 nm, supplied by io-li-tec nanomaterials.
- NTI-4: Titanium dioxide (TiO2) 99% covered with silicone dioxide (SiO2), particle size: 20-40 nm, supplied by io-li-tec nanomaterials.
- NTI-5: Rutile titanium dioxide (TiO2), purity 99.5% with traces of basic metals, particle size <100 nm, supplied by io-li-tec nanomaterials.

The results of this experiment are shown in Table 3.

**Table 3. Results of the polymerization in the presence of different inorganic nanoparticles based on TiO₂.**

| Nanoparticles Inorganic | Concentration (mg/mL) | Polymeric particle size obtained (nm) | Polydispersity index PDI | Reaction yield (%) |
|---|---|---|---|---|
| NT11 | 0.0225 | 2318 ± 45 | 0.079 ± 0.052 | 63 ± 10 |
| NT12 | 0.0513 | 902 ± 30 | 0.087 ± 0.032 | 60 ± 9 |
| NT13 | 0.0525 | 967 ± 32 | 0.199 ± 0.100 | 61 ± 10 |
| NTI4 | 0.0538 | 805 ± 25 | 0.081 ± 0.040 | 63 ± 10 |
| NTI5 | 0.0465 | 1264 ± 47 | 0.055 ± 0.028 | 62 ± 11 |

As is shown in Table 3, at the concentrations of inorganic nanoparticles used, the sizes of the polymeric particles obtained are greater than 300 nm.

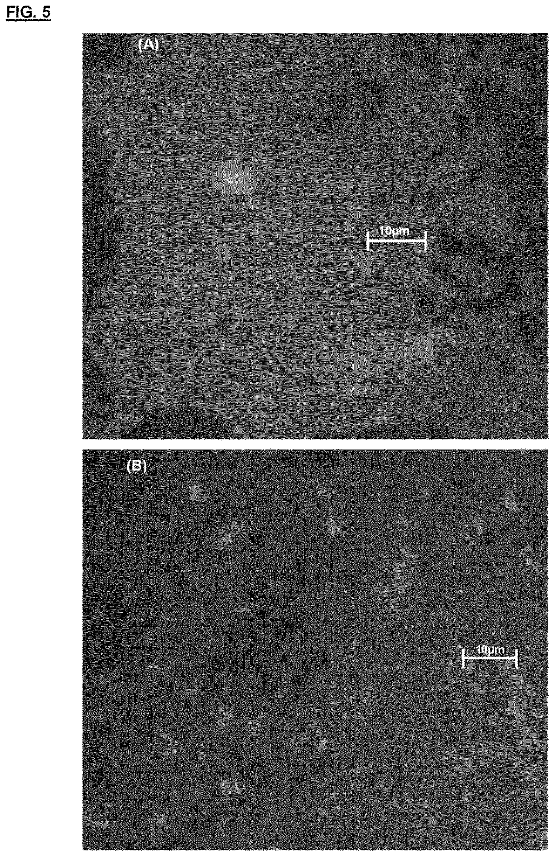

Fig. 5 shows optical microscope images of the polymeric particles synthesized in the presence of the inorganic nanoparticles NTI4 (A) and NTI5 (B), where it can be seen how said polymeric particles have a high monodispersibility and compact bundling. The sample comprising inorganic nanoparticles NTI4 corresponds to TiO₂ nanoparticles covered with SiO₂.

The following experiment consisted of testing the effect of the concentration of inorganic nanoparticles on the particle size. The results are shown in Table 4.

**Table 4. Polymeric particles size as a function of the nature of the inorganic particle and the concentration of said inorganic nanoparticles in the precipitation polymerization reaction.**

| PRECIPITATION POLYMERIZATION IN THE PRESENCE OF NTI-1 | | | | |
|---|---|---|---|---|
| Nanoparticles Inorganic | Concentration (mg/mL) | Polymeric particle size (nm) | Polydispersity index PDI | Yield (%) |
| NTI1-A | 0.023 | 2150 ± 22 | 0.080 ± 0.050 | 65 ± 10 |
| NTI1-B | 0.112 | 1825 ± 32 | 0.123 ± 0.080 | 65 ± 9 |
| NTI1-C | 0.145 | 1720 ± 25 | 0.056 ± 0.042 | 65 ± 10 |
| NTI1-D | 0.210 | 1750 ± 42 | 0.140 ± 0.085 | 66 ± 10 |
| NTI1-E | 0.320 | 1500 ± 52 | 0.127 ± 0.100 | 63 ± 11 |
| NTI1-F | 0.408 | 1550 ± 28 | 0.095 ± 0.042 | 65 ± 12 |
| NTI1-G | 0.523 | 1420 ± 35 | 0.120 ± 0.070 | 65 ± 10 |
| NTI1-H | 0.640 | 1324 ± 40 | 0.086 ± 0.023 | 62 ± 10 |

| PRECIPITATION POLYMERIZATION IN THE PRESENCE OF NTI-2 | | | | |
|---|---|---|---|---|
| Nanoparticles Inorganic | Concentration (mg/mL) | Polymeric particle size (nm) | Polydispersity index PDI | Yield (%) |
| NTI2-A | 0.023 | 810 ± 15 | 0.085 ± 0.052 | 65 ± 10 |
| NTI2-B | 0.103 | 828 ± 23 | 0.145 ± 0.096 | 64 ± 12 |
| NTI2-C | 0.154 | 809 ± 30 | 0.026 ± 0.010 | 64 ± 10 |
| NTI2-D | 0.205 | 833 ± 42 | 0.030 ± 0.020 | 64 ± 10 |
| NTI2-E | 0.310 | 1021 ± 41 | 0.098 ± 0.005 | 61 ± 9 |
| NTI3-F | 0.410 | 1058 ± 28 | 0.123 ± 0.067 | 64 ± 10 |
| NTI3-G | 0.542 | 1123 ± 33 | 0.130 ± 0.096 | 63 ± 10 |
| NTI3-H | 0.650 | 1210 ± 58 | 0.120 ± 0.052 | 65 ± 10 |

| PRECIPITATION POLYMERIZATION IN THE PRESENCE OF NTI-3 | | | | |
|---|---|---|---|---|
| Nanoparticles Inorganic | Concentration (mg/mL) | Polymeric particle size (nm) | Polydispersity index PDI | Yield (%) |
| NTI3-A | 0.025 | 1825 ± 60 | 0.063 ± 0.052 | 69 ± 10 |
| NTI3-B | 0.110 | 1735 ± 45 | 0.122 ± 0.095 | 65 ± 12 |
| NTI3-C | 0.150 | 1523 ± 52 | 0.085 ± 0.023 | 65 ± 10 |
| NTI3-D | 0.210 | 1350 ± 25 | 0.123 ± 0.094 | 68 ± 7 |
| NTI3-E | 0.320 | 1148 ± 39 | 0.145 ± 0.100 | 62 ± 10 |
| NTI3-F | 0.420 | 1129 ± 58 | 0.110 ± 0.095 | 63 ± 11 |
| NTI3-G | 0.528 | 1123 ± 24 | 0.096 ± 0.071 | 65 ± 9 |
| NTI3-H | 0.635 | 1138 ± 36 | 0.085 ± 0.006 | 63 ± 10 |

| PRECIPITATION POLYMERIZATION IN THE PRESENCE OF NTI-4 | | | | |
|---|---|---|---|---|
| Nanoparticles Inorganic | Concentration (mg/mL) | Polymeric particle size (nm) | Polydispersity index PDI | Yield (%) |
| NTI4-A | 0.027 | 896 ± 25 | 0.060 ± 0.035 | 66 ± 10 |
| NTI4-B | 0.108 | 693 ± 22 | 0.063 ± 0.023 | 64 ± 12 |
| NTI4-C | 0.161 | 482 ± 15 | 0.035 ± 0.012 | 64 ± 10 |
| NTI4-D | 0.215 | 435 ± 32 | 0.002 ± 0.001 | 64 ± 9 |
| NTI4-E | 0.323 | 368 ± 42 | 0.007 ± 0.003 | 63 ± 10 |
| NTI4-F | 0.430 | 328 ± 28 | 0.004 ± 0.002 | 63 ± 10 |
| NTI4-G | 0.538 | 280 ± 31 | 0.063 ± 0.023 | 62 ± 11 |
| NTI4-H | 0.645 | 233 ± 19 | 0.095 ± 0.0082 | 62 ± 10 |

In light of the results obtained, it can be seen that only when inorganic nanoparticles NTI4 are used at concentrations below 0.430 mg/mL (0.053% by weight with respect to the total weight of the solution) are polymeric particles with sizes less than 300 nm obtained. Note that the inorganic nanoparticles NTI4 are precisely the TiO₂ nanoparticles covered with SiO₂, therefore, we can confirm that the SiO₂ nanometric nuclei are responsible for the reduction of the polymeric particle size and, in turn, the decrease of the polydispersity index values. We can conclude that the lixiviated nuclei are mainly SiO₂ nanometric nuclei.

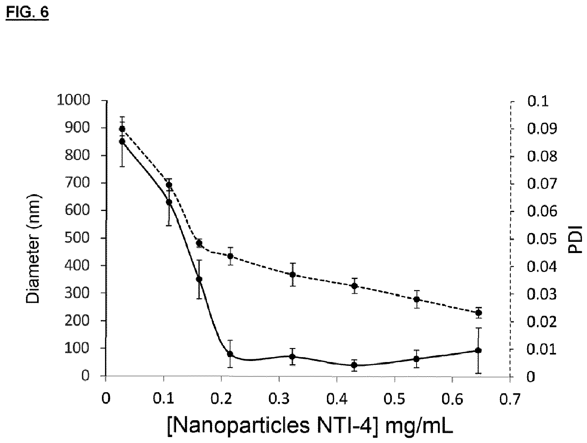

In Fig. 6, it can be seen how the size of the polymeric particles and the polydispersity index (PDI) values vs. concentration of inorganic nanoparticles NTI4 present in the polymerization medium decrease.

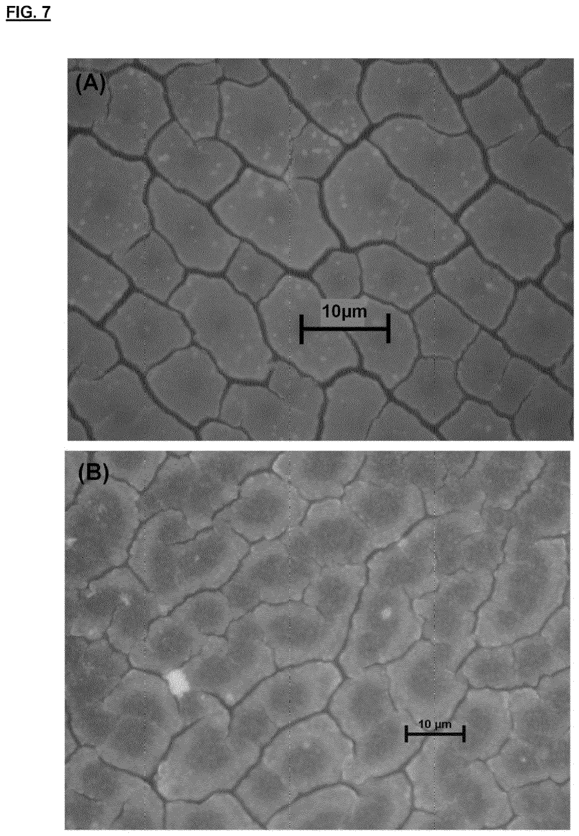

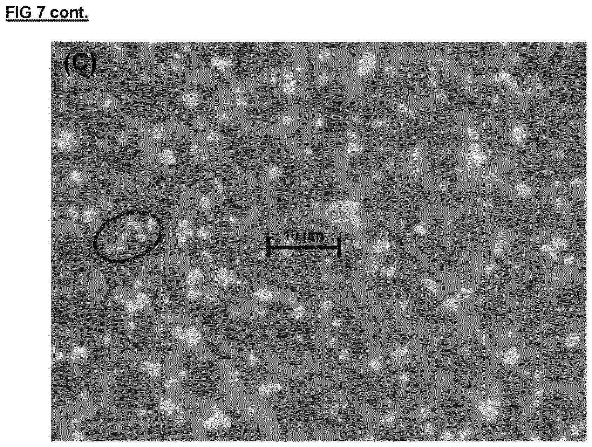

Fig. 7 shows optical microscope images of the polymeric particles obtained using the inorganic nanoparticles NTI4-F (A), NTI4-G (B) and NTI4-H (C). Highly monodisperse polymeric particles in the form of a compact bundling are observed.

In addition, in Fig. 7, it can be seen that the optimal concentration of inorganic nanoparticles NTI4 is from 0.538 mg/ml (NTI4-G) resulting in a polymeric particle size of 280 ± 31 nm; based on said concentration, an excess of TiO₂ inorganic nanoparticles covered with SiO₂ can be observed which are deposited on the surface of the polymeric particles which they form.

The SiO₂ nuclei coming from the glass are highly porous, hydrophilic inorganic materials with increased specific surface and considered fractal surfaces. Said SiO₂ nuclei have a high affinity for the polymers used in this synthesis; the strength of the SiO₂ polymer interactions are much greater than the polymer-polymer, polymer-solvent and solvent-SiO₂ interactions, which reduces the energy barrier of the nucleation due to the surface energy almost to zero which would mean that the phase separations are only produced on the surface of these SiO₂ primary nuclei coming from the glass. Therefore, if said SiO₂ nuclei are maintained at a suitable concentration in the reaction medium, when the polymeric chains reach the maximum molecular weight which can be produced in solution, the phase separation mainly takes place on the SiO₂ nuclei, thus drastically reducing the particle size; the polymeric particle size obtained is thereby much smaller and the resulting polymeric particles are highly monodisperse (see Fig. 1). The particle size is reduced up to 4 times with respect to the particle size obtained in precipitation polymerization in the absence of SiO₂ nuclei (see Figures 3, 4 and 6). We can conclude that by controlling the concentration of SiO₂ nuclei in suspension we can modulate the polymeric particle size and obtain monodisperse particle populations.

In addition, if we compare the values of the polydispersity index obtained in the precipitation polymerization in the presence and absence of SiO₂ nuclei, it can be concluded that the values of the polydispersity index in the presence of SiO₂ nuclei are very low.

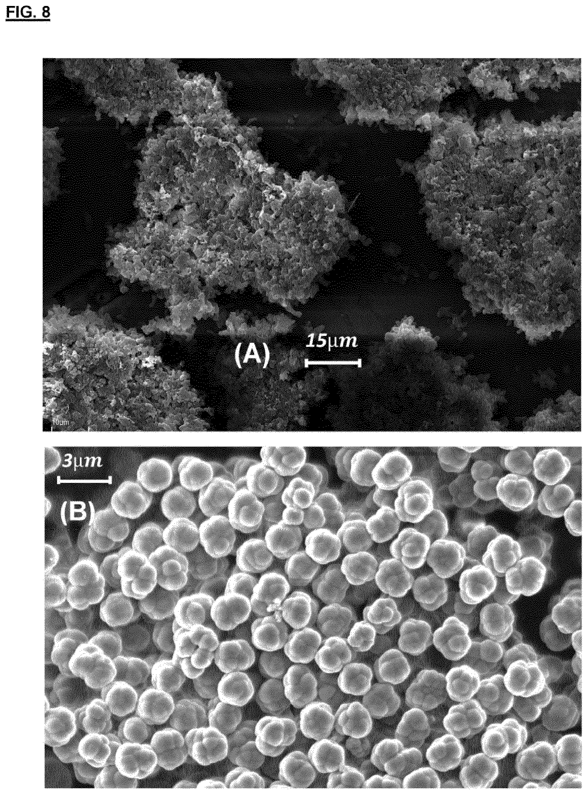

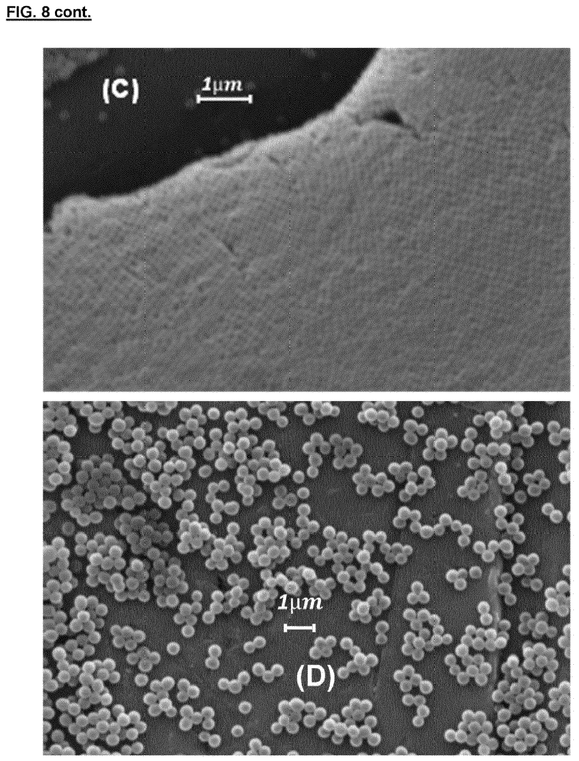

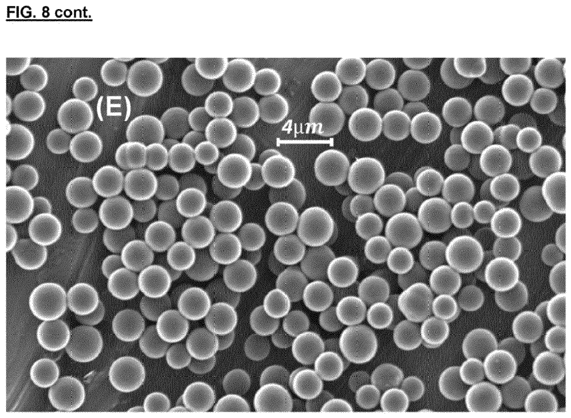

In addition, Fig. 8 shows how when the precipitation polymerization is carried out in different solvents or mixtures thereof, the particle size changes depending on the type of solvent/mixture used. If the polymerization reaction is carried out in pure solvents with a high value of the Hansen solubility parameter to form a hydrogen bridge (δₕ) such as alcohols, the polymeric chains of the copolymer are maintained solvated up to high conversion values, amorphous macrogels being obtained; if the polymerization reaction is carried out in pure solvents with a high value of the Hansen solubility parameter to provide dispersive strengths (δ_{d}) such as apolar solvents (toluene, xylene, ethylbenzene, octane, heptane, hexane, etc.), 1500 nm diameter non-spherical particles formed by aggregation/coagulation of smaller particles are obtained (see Fig. 8); if the polymerization reaction is carried out in pure solvents with an increased value of the Hansen solubility parameter to provide polar interactions (δ_{ρ}) (such as acetonitrile, acrylonitrile, propionitrile, benzonitrile, butyronitrile, etc.) particles of between 250 and 500 nm are obtained; if the polymerization reaction is carried out in mixtures (e.g. acetonitrile/2-propanol/toluene) of the three types of solvents in which the value of each one of the Hansen solubility parameters can be modulated preferably in mixtures of acetonitrile/2-propanol/toluene, particles sizes of between 600 nm and 2500 nm can be obtained.

To functionalize with Zn⁺², Ca⁺² or doxycycline, the polymeric particles obtained in the presence of SiO₂ nuclei are suspended in aqueous solutions of ZnCl₂, CaCl₂ and/or doxycycline, are maintained under agitation for the time required so that the functionalization is produced, which can be from a few hours to days: time required for reaching the adsorption balance). They are then centrifuged and dried in a vacuum oven to constant weight. Specific examples of polymeric particles functionalized with Zn⁺², Ca⁺² or doxycycline are shown below.

### EXAMPLE 2: Polymeric particles functionalized with doxycycline to prevent or treat infectious recurrences after endodontic interventions

The polymeric particles functionalized with doxycycline can be obtained by functionalizing the polymeric particles previously obtained with a broad spectrum antibiotic, effective against bacteria commonly found in root canals (doxycycline). The polymeric particles functionalized with doxycycline are capable of slowly releasing the antibiotic over more than 28 days and at an effective dose as is shown below:
To functionalize with doxycycline, the spherical particles with size less than 300 nm obtained previously in the presence of SiO₂ nuclei, the following protocol is followed:
30 mg of polymeric particles with size less than 300 nm were submerged in 18 mL of aqueous solution of doxycycline hyclate (Sigma-Aldrich, Darmstadt, Germany) at a concentration of 40 mg/mL, over 4 hours, under continuous agitation. The suspensions were then centrifuged, the particles were separated from the supernatant and were dried in a vacuum oven to constant weight.

The quantity of doxycycline in the aqueous solution before the immersion of the polymeric particles with size less than 300 nm and in the supernatant, after the incubation of the polymeric particles in the solution of doxycycline was analyzed by high resolution liquid chromatography (HPLC) (Waters Alliance 2690, Waters Corporation, Milford, MA, USA), technology equipped with a UV-Vis detector. A binary mobile phase was used formed by two solvents A and B in the manner of an isocratic elution with a proportion of 80:20 (A:B). The mobile phase A was an aqueous solution of KHPO₄ 50 mM and the mobile phase B 100% acetonitrile. The flow used was from 1.0 ml/min and the total analysis time was 10 min. The retention time was 4.85 min. The doxycycline concentration was calculated based on a standard curve with known levels of doxycycline, measuring the absorbency at 273 nm.

The release profile of doxycycline coming from the polymeric particles functionalized with doxycycline was evaluated after incubating polymeric particles functionalized with doxycycline at 37°C in 1 mL of saline solution buffered with phosphate buffer (pH= 7.4). After the incubation process, 100 µL aliquots were collected at the time intervals: 12 hours, 24 hours, 48 hours, 7 days, 14 days, 21 days and 28 days. After each supernatant collection process, fresh buffer was added to the polymeric particle suspensions with size less than 300 nm. The aliquots were stored at -20°C until analysis. All the experiments were carried out three times.

The quantity of doxycycline found in the solution after the immersion and incubation of the polymeric particles functionalized with doxycycline was 399.5 µg/ml (per mg of NP) which shows load efficacy of approximately 70%.

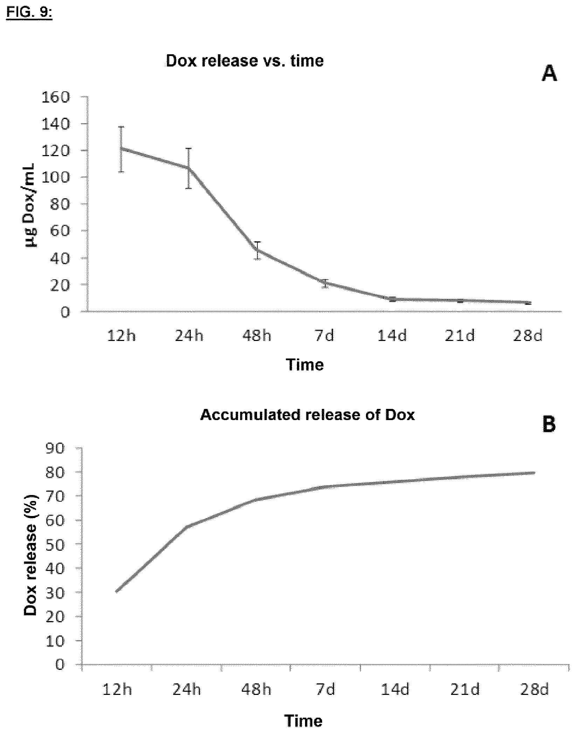

In Fig. 9A, the release values of doxycycline (in µg/ml) are shown for each mg of polymeric particles functionalized with doxycycline for each time interval studied (average values and standard deviation).

At 12 hours, the doxycycline release was 121 µg/ml (per mg of polymeric particles functionalized with doxycycline). A rapid release effect of doxycycline at greater doses was observed from 12 hours to the first week of storage.

After 7 hours, the antibiotic release was slower and greater than 20 µg/ml (per mg of polymeric particles functionalized with doxycycline).

After 14 days, the doxycycline release was practically maintained stable, being 9, 8 and 6 µg/ml (per mg of polymeric particles functionalized with doxycycline) at 14, 21 and 28 days, respectively.

Fig. 9B shows the accumulated release of doxycycline over 28 days.

According to the data, the doxycycline is released sustainably at least over 28 days, with a rapid release effect at 24 hours. For any time period of the studies, the dose released is much greater than that considered effective against *Porphyromonas gingivalis (Pg).*

Table 6 shows all the data of doxycycline release: release of doxycycline (µg/mL per mg of polymeric particles functionalized with doxycycline) at each time point, accumulated release of doxycycline in each time frame (µg/mL per mg of polymeric particles functionalized with doxycycline) and also, the accumulated release in %.

**Table 6: Doxycycline release data per mg of polymeric particles functionalized with doxycycline.**

| Time | Release (µg dox/mL) | Accumulated release (µg dox/mL) | Accumulated release (%) |
|---|---|---|---|
| 12 h | 121.129 | 121.129 | 30 |
| 24 h | 106.598 | 227.727 | 57 |
| 48 h | 45.808 | 273.535 | 68 |
| 7 d | 21.0805 | 294.6155 | 74 |
| 14 d | 9.1065 | 303.722 | 76 |
| 21 d | 8.185 | 311.907 | 78 |
| 28 d | 6.323 | 318.23 | 80 |

The average bacterial susceptibility to doxycycline is around 0.1 to 0.2 µg/mL and the minimum inhibitory concentration (MIC) to which *PG* is susceptible is 6 µg/mL. Doxycycline acts against PG inhibiting the synthesis of various microbial proteins. Doxycycline is bonded to ribosome to avoid the synthesis of RNA since it prevents the addition of more amino acids to the polypeptide. Doxycycline also causes a powerful and lasting inhibition of the metalloproteases of the dentin matrix which are related to chronic inflammation processes and the abysses at apicial level. Therefore, the long-term administration of a subantimicrobial dose of doxycycline can be considered an effective treatment for periodontal and periapical inflammation even when it does not induce antimicrobial effects on the subgingival microflora.

The levels of doxycycline release found can have antibacterial effects not only for *Pg,* but also for other bacteria present in the human subgingival plaque. Doxycycline at a concentration of between 0.5 and 1 µg/mL is bactericidal against different *Pg* strains and at a concentration of between 0.1 and 6.0 µg/mL it is effective against putative periodontal pathogens. It is important to point out that the evaluated polymeric particles functionalized with doxycycline are capable of releasing more than 6.2 µg/ml even after 28 days.

The data herein outlined in terms of doxycycline release shows a sustained release and much more effective than other release systems tested up to now, such as for example a doxycycline formulation incorporated in a cellulose acetate matrix studied by Tonetti [Tonetti MS, Lang NP, Cortellini P, Suvan JE, Eickholz P, Fourmousis I, Topoll H, Vangsted T, Wallkamm B. Effects of a single topical doxycycline administration adjunctive to mechanical debridement in patients with persistent/recurrent periodontitis but acceptable oral hygiene during supportive periodontal therapy. J Clin Periodontol 2012;39(5):475-482].

In the work by Kim et al. [Kim TS, Bürklin T, Schacher B, Ratka-Krüger P, Schaecken MT, Renggli HH, Fiehn W, Eickholz P. Pharmacokinetic profile of a locally administered doxycycline gel in crevicular fluid, blood, and saliva. J Periodontol 2002;73(11):1285-1291], the formulation of a biodegradable doxycycline gel and a release capacity of 20 mg/mL of doxycycline at 15 minutes which is reduced to 577 µg/mL after 3 days and to 16 µg/mL at 12 days are described.

In [Deasy PB, Collins AE, MacCarthy DJ, Russell RJ. Use of strips containing tetracycline hydrochloride or metronidazole for the treatment of advanced periodontal disease. J Pharm Pharmacol 1989;41(10):694-699], the use of tetracycline hydrochloride together with poly(hydroxybutyric acid) to produce a biodegradable polymeric matrix is described, which showed a sustained release for slightly more than 5 days, with a rapid release effect at a very significant high dose during the first 24 hours.

In general, all the materials previously published can release doxycycline in greater concentrations but in very short periods of time which denotes uncontrolled rapid release effects in very short time. The polymeric particles functionalized with doxycycline (polymeric particles functionalized with doxycycline) are capable of slowly releasing the antibiotic over more than 28 days and at an effective dose.

The experiment carried out to show the antibacterial efficiency of polymeric particles functionalized with doxycycline (polymeric particles functionalized with doxycycline) against *Pg* in planktonic culture is described below.

The bacteria Pg 33277 were obtained from ATCC (Bethesda, MD). The anaerobic microorganism *Pg* was cultivated in a tryptic soy medium supplemented with yeast extract (5 g/L), hemin (5 mg/L), menadione (1 mg/L) in an anaerobic recipient (80% N₂, 10% CO₂, 10% H₂) over 72 hours. The bacteria were collected by centrifugation and resuspended in the same culture medium. The number of bacteria per mL was determined by measuring the optical density at 600 nm and adjusting it to a standard bacteria suspension of 1x10⁷ CFU/ml.

The polymeric particles functionalized with doxycycline and a control formed by polymeric particles without functionalization were suspended in saline phosphate buffer at three different concentrations (10 mg/ml, 1 mg/ml and 0.1 mg/ml). The suspensions were placed in Eppendorf tubes with bacteria cultures (1x10⁷ CFU/ml for each 0.45 ml of suspension) and were incubated for 3, 12, 24 and 48 hours at 37°C. At the end of each incubation period, the effect on the bacteria was evaluated and is described below. All the experiments were carried out in anaerobiosis conditions and the viability of the bacterial cells was evaluated determining the capacity thereof to transform a tetrazolium salt (3-[4,5-dimethylthiazol-2-yl] -2,5-diphenyl tetrazolium bromide) (MTT) in the colorant formazan (Sigma Chemical Co.). The different bacterial suspensions were placed in a 96-well microplate. The MTT marking reactive was added to each culture well and the plates were incubated for 4 h at 37°C. After incubation, a solubilizing reactive was added, provided by the manufacturer and then incubated again over night at room temperature.

In order to evaluate the number of viable bacterial cells, a spectroscopic determination was carried out of the purple color coming from the colorant formazan produced from MTT by the viable bacterial cells. To this end, readings were taken at 560 nm, using an ELISA reader (Spectrostar Nano, BMG Labtech, Cary, NC, USA.) [Banzi et al., 2014]. As a control parameter, an incubated bacteria culture was used without the presence of particles only in phosphate buffer.

All the tests were carried out three times. The data were expressed as average ± standard deviation and were analyzed by means of ANOVA analysis and the Scheffe F method for post hoc comparisons.

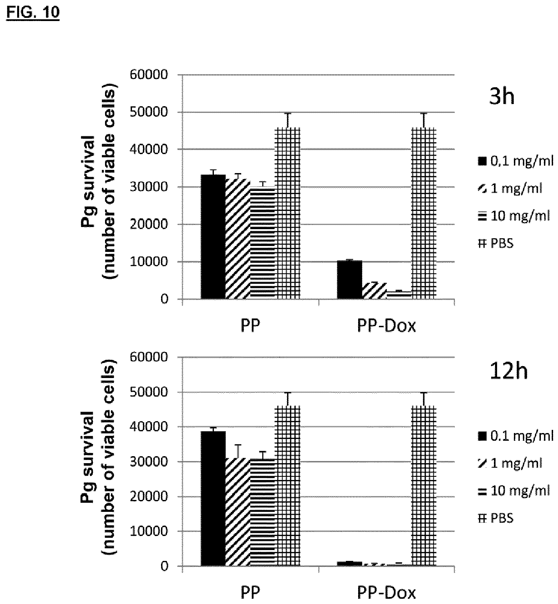

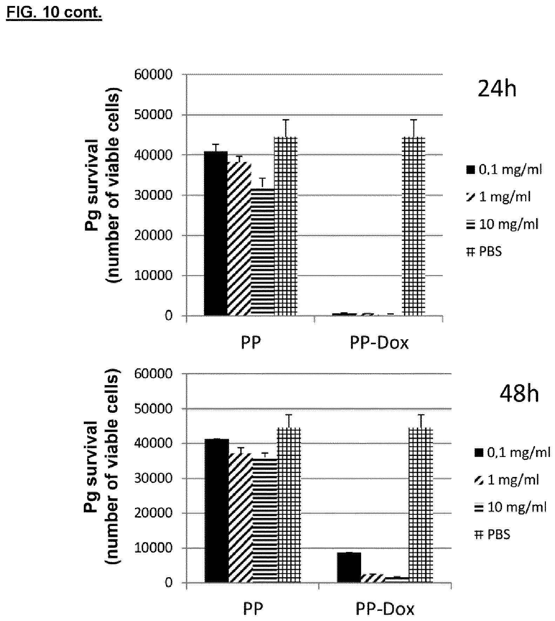

In Fig. 10, the survival values are shown expressed as numbers of viable Pg cells following incubation after 3, 12, 24 and 48 h of exposure to polymeric particles functionalized with doxycycline, unfunctionalized polymeric particles and phosphate buffer as control.

The polymeric particles functionalized with doxycycline showed a bacterial reduction of between 77.5 to 98.8% in the different concentrations and incubation times tested. At 24 hours, all the concentrations tested of polymeric particles functionalized with doxycycline reached more than 98% of bacterial death. After 48 hours of incubation, these values were reduced to 80.3%, 94.8% and 96.4% (concentrations of 0.1, 1 and 10 mg/ml respectively).

The advantage of the polymeric particles functionalized with doxycycline herein described is the long-lasting release effect of the antibacterial and the association of two beneficial effects: 1) antibacterial effect 2) inhibition of the metalloproteases. It is a simple formulation to apply and is compatible with all the materials used for filling the root canal. It is expected that the application of this formulation in the root canal reduces/prevents/resolves, to a large extent, the infectious recurrences and the need for secondary surgical interventions subsequent to the endodontic interventions. Since the biocompatibility of the polymeric particles functionalized with doxycycline is greater than 90% with human fibroblasts, these polymeric particles functionalized with doxycycline may be a very useful tool when endodontic revascularization techniques are used. An additional application of these particles is the topical administration in the dentin. They can close the dentin tubules, inhibit the metalloproteases of the dentin and restore the mechanical properties of the dentin interface.

### EXAMPLE 3: Polymeric particles functionalized with zinc and with calcium for the treatment of dentin hypersensitivity

The polymeric spherical particles functionalized with Ca⁺² and Zn⁺² are capable of being administered topically on the surface of the dentin, they are bonded to proteins of the dentin matrix (collagen) and facilitate the remineralization thereof by way of the slow release of said ions and the attraction of calcium and phosphate which contain the biological fluids.

The polymeric spherical particles functionalized with Ca⁺² and Zn⁺² were obtained in the following manner: 30 mg of polymeric particles were submerged at room temperature for 3 days under continuous agitation in aqueous solutions of ZnCl₂ or CaCl₂, with zinc or calcium concentrations of 40 mg/L, at pH 6.5 with the aim of achieving the adsorption balance of the metallic ions. The suspensions were then centrifuged and the particles were separated from the supernatant and were dried in a vacuum oven to constant weight. The values achieved in the ion bond were 0.96 ± 0.04 µg particles Ca/mg and 2.15 ± 0.05 µg particles Zn/mg. The release capacity of the calcium and zinc ions was measured after incubating the polymeric spherical particles functionalized with Ca⁺² and Zn⁺² in 5 mL of bidistilled water at 37°C. After this incubation step, 5 mL aliquots were collected at the following time intervals: 24 hours, 48 hours, 7 days, 21 days 28 days. After each collection of supernatant, 5 mL of fresh bidistilled water was added. The aliquots were stored at -20°C until the analysis thereof. The concentrations of calcium and zinc were analyzed by way of a spectrometer (ICP-OES) (ICP-OES Optima 8300, Perkin-Elmer, MA, USA.). All the tests were carried out three times.

The release results (average and standard deviation) of the Ca²⁺ and Zn²⁺ ions are shown in Table 7, the values expressed as µg of ion released per 10 mg of polymeric spherical particles functionalized with Ca⁺² and Zn⁺² at each time point.

The calcium release varied from 0.856 to 1.007 µg over the first week. This calcium release doubled after 21 days, being around 2 µg The polymeric spherical particles functionalized with Zn⁺² maintained a sustained zinc release which varied from 0.021 to 0.025 µg between 12 hours and 21 days. A double increase was observed in day 28 when 0.044 µg was released.

Taking into account the initial cation load for every 10 mg of polymeric particles functionalized with Ca⁺²: 9.6 ± 0.4 µg Ca²⁺ and polymeric particles functionalized with Zn⁺²: 21.5 ± 0.5 µg Zn²⁺), the total release percentages of Ca²⁺ and Zn²⁺ for every 10 mg of particles at each time period is also shown in Table 7.

**Table 7: Average values and standard deviations of the µg of Ca²⁺ and Zn^{2 +}ions released for every 10 mg of particles as a function of the time and % of accumulated release of each ion for every 10 mg of particles**

| Time | Ca²⁺ (µg) | % Release accumulated Ca²⁺ | Zn²⁺ (µg) | % Release accumulated Zn²⁺ |
|---|---|---|---|---|
| 12 h | 1.006 (0.002) | 10.5 | 0.025 (0.001) | 0.1 |
| 24 h | 1.007 (0.001) | 21.0 | 0.025 (0.001) | 0.2 |
| 48 h | 0.909 (0.003) | 30.4 | 0.023 (0.002) | 0.3 |
| 7 d | 0.856 (0.001) | 39.4 | 0.021 (0.001) | 0.4 |
| 21 d | 2.082 (0.05) | 61.0 | 0.024 (0.002) | 0.5 |
| 28 d | 2.031 (0.02) | 82.2 | 0.044 (0.005) | 0.8 |

| Time | Ca²⁺ (µg) | % Release accumulated Ca²⁺ | Zn²⁺ (µg) | % Release accumulated Zn²⁺ |
|---|---|---|---|---|
| 12 h | 1.006 (0.002) | 10.5 | 0.025 (0.001) | 0.1 |
| 24 h | 1.007 (0.001) | 21.0 | 0.025 (0.001) | 0.2 |
| 48 h | 0.909 (0.003) | 30.4 | 0.023 (0.002) | 0.3 |
| 7 d | 0.856 (0.001) | 39.4 | 0.021 (0.001) | 0.4 |
| 21 d | 2.082 (0.05) | 61.0 | 0.024 (0.002) | 0.5 |
| 28 d | 2.031 (0.02) | 82.2 | 0.044 (0.005) | 0.8 |

The polymeric spherical particles functionalized with Ca⁺² and Zn⁺² can occlude the dentin tubules. This occlusion can reduce the pain when there is dentin hypersensitivity. The occlusion capacity of the tubules has been demonstrated by means of direct observation with scanning electron microscopy and permeability tests through the dentin.

To verify the occlusion of the dentin tubules, the following treatment protocol was carried out: Healthy molars were obtained with the informed consent of the donors (from 18 to 25 years of age), according to a protocol approved by the Research Ethics Committee of the Institution (405/CEIH/2017), the dentin samples were exposed to a solution of citric acid (pH 3.8) for 1 minute to guarantee the initial opening of the dentin tubules and eliminate the smear layer. The dentin surfaces were rinsed before applying the functionalized and unfunctionalized polymeric particles. Suspensions in phosphate buffer of unfunctionalized polymeric particles, the polymeric spherical particles functionalized with Ca⁺² and Zn⁺² at a concentration of 10 mg/mL) and a phosphate buffer solution without any kind of particles, which is termed untreated dentin, were applied for 30 seconds. Said samples are stored in phosphate buffer for 24 hours and 7 days.

The samples were then incorporated into a 2.5% glutaraldehyde solution in a 0.1 M sodium cacodylate buffer for 24 h. The samples were subjected to supercritical drying (Leica EM CPD 300, Vienna, Austria), they were covered with carbon by means of a Nanotech Polaron-SEMPREP2 sputter coating (Polaron Equipment Ltd., Watford, UK) and were evaluated by means of scanning electron microscope FESEM (FESEM Gemini, Carl Zeiss, Oberkochen, Germany).

The tubular occlusion percentage was determined from the FESEM topographic images, the following states of the tubules being defined as the possible stages: "open" tubules (A), "partially filled" tubules (P), occluded tubules (O) and "covered" tubules (C).

The analysis of disperse energy was carried out at selected points using, to this end, an R-X detector system (EDX Inca 300, Oxford Instruments, Oxford, United Kingdom) coupled with the FESEM.

As can be observed in Fig. 11A, 100% of the dentin tubules were open in untreated dentin, after 7 days of storage. At 24 hours, 100% of the tubules were shown occluded in the treated cervical dentin with unfunctionalized polymeric particles. Half of these tubules remained full after 7 days of storage.

In Fig. 11B, it can be observed how there are 100% of closed tubules in dentin samples treated with spherical polymeric particles functionalized with Zn⁺² (after 7 days of application). In the case of polymeric spherical particles functionalized with Ca⁺², after 7 days, some tubules started to be visible, a large quantity of them (95%) remaining occupied. The occlusion of the dentin tubules was due to the deposit and growth of mineral structures composed of calcium and phosphate which was revealed with the detection of Ca and P in the qualitative analysis.

Table 8 shows all the data of the state of the tubule canals at 24 hours of the treatment of the dentin surfaces with the different formulations used and after 7 days of applied treatment. While the mineral deposits generated after the application of the particles seems not to disappear with time, the sodium and calcium phosphosilicates which form carbonated apatites used in other treatments of the hypersensitivity of the dentin show high rates of solubility. Similarly, the different calcium, potassium or ferrite oxalates also used to treat the hypersensitivity of the dentin show high rates of elimination or dissolution by action of saliva.

Another treatment, recently proposed, for dentin hypersensitivity is the application of nanohydroxyapatite particles because they cause closure of tubules, but it has been demonstrated that these buffers are dissolved and compromise the efficacy of the treatment in the long term.

**Table 8: Percentage of occluded tubules as a function of the polymeric spherical particles functionalized with Ca⁺² and Zn⁺² abbreviations. Untreated: untreated dentin, A: open, P: partially full, O: occluded, C: covered.**

| | 24 h | | | | | 7 d | | | | |
|---|---|---|---|---|---|---|---|---|---|---|
| | O | P | F | C | Total | O | P | F | C | Total |
| Untreated | 90 | 10 | 0 | 0 | 100 | 80 | 20 | 0 | 0 | 100 |
| Polymeric particle | 0 | 0 | 100 | 0 | 100 | 10 | 20 | 50 | 20 | 100 |
| Polymeric particle functionalized with Zn⁺² | 40 | 10 | 20 | 30 | 100 | 0 | 0 | 0 | 100 | 100 |
| Polymeric particle functionalized with Ca⁺² | 0 | 0 | 0 | 100 | 100 | 0 | 5 | 95 | 0 | 100 |

To evaluate the permeability of the dentin, different healthy uniradicular teeth were obtained with the informed consent of the donors (from 18 to 25 years of age), according to a protocol approved by the Research Ethics Committee of the Institution (405/CEIH/2017). 5 mm of each root was eliminated, below the cement-enamel bond, with the aid of a diamond blade mounted on a microtome at low speed (Accutom-50 Struers, Copenhagen, Denmark). A fluid filtration system was inserted in the pulpar chamber to measure the volume of fluid which can pass through the dentin. A cervical cavity was prepared in the shape of a V with a mesial-distal width of 5 mm and an occlusal cervical height of 3 mm and a depth of 2 mm with an 801-014 carbide bur. Acid etching (32% phosphoric acid) was carried out to remove the smear layer and to ensure dentin permeability. Suspensions in phosphate buffer of unfunctionalized polymeric particles, polymeric particles functionalized with Ca⁺² and Zn⁺², at a concentration of 10 mg/mL or only one solution of buffer (for the test of the untreated dentin) were applied. After the treatment, the samples were stored for 24 hours and 7 days. The dentin fluid flow (DFF) through the cervical dentin was measured after the two storage periods. 5 fluid volume readings were registered every 3 minutes for 15 minutes and the average was calculated. The rate of the dentin fluid flow DFF was evaluated after the application of the different treatments, with respect to the initial flow rate [% of reduction in the flow rate = 100 x (DFFᵢₙᵢₜᵢₐₗ- DFFₚₒₛₜ₋ₜᵣₑₐₜₘₑₙₜ) / DFFᵢₙᵢₜᵢₐₗ]. The statistical analysis was carried out with multiple comparative ANOVA and Student Newman Keuls samples. A value of P < 0.05 was established to obtain significance.

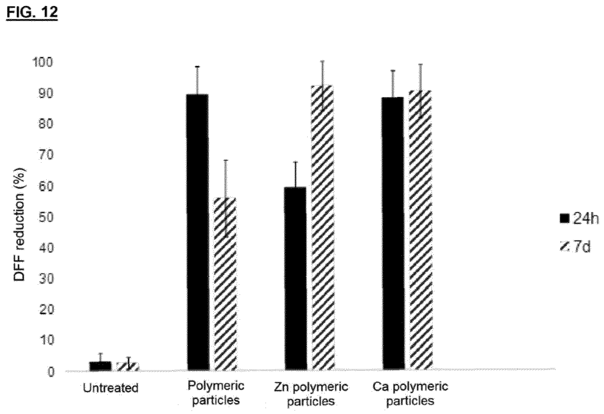

In Fig. 12, the % of decrease of dentin fluid flow DFF in each sample can be observed.

At 24 hours, the cervical dentin treated both with unfunctionalized polymeric particles and with polymeric particles functionalized with Ca⁺² obtained the greatest reduction of the dentin fluid flow DFF.

At 7 days of treatment, the dentin treated both with polymeric spherical particles functionalized with Ca⁺² and Zn⁺² achieved the greatest reduction of dentin fluid flow DFF.

The samples treated with polymeric spherical particles functionalized with Zn⁺² showed a reduction of dentin fluid flow DFF greater than 90% after 7 days of storage and the dentin treated with polymeric spherical particles functionalized with Ca⁺² did not modify the dentin fluid flow DFF values during the periods studied.

A reduction in the flow index unequivocally reflects the effect of the occlusion of the dentin tubules and the disappearance of the pain in the patients.

The presence of 30% of the dentin tubules open or partially full (Table 2) show a reduced occlusion capacity and a temporary remineralizing effect of the unfunctionalized polymeric particles. In addition, the low reduction of the fluid flow (Fig. 12) at 7 days disregarded the use of unfunctionalized polymeric particles for the hypersensitivity of the dentin.

On the contrary, when the dentin was treated with polymeric particles functionalized with Ca⁺² and Zn⁺², the reduction of the dentin fluid flow DFF at 7 days of storage achieved similar values, close to 90% (Fig. 2). This substantial effect on the reduction in the dentin fluid flow index is associated with the occlusion of the dentin tubules, owing to the generation of calcium phosphate salt deposits, as confirmed after the analysis with FESEM and EDX. Therefore, polymeric particles functionalized with Ca⁺² and Zn⁺² have demonstrated a high capacity for the occlusion of the dentin tubules and should be considered a viable treatment for the hypersensitivity of the dentin since they can occlude the dentin tubules when calcium phosphate precipitation is induced.

To evaluate the nanohardness (Hi) and Young's elasticity modulus, healthy teeth were obtained with the informed consent of the donors (from 18 to 25 years of age), according to a protocol approved by the Research Ethics Committee of the Institution (405/CEIH/2017). Two blocks of dentin were obtained from the buccal surfaces of each root, just below the cement-dentin bond by means of cutting using a diamond blade mounted on a microtome (Accutom-50 Struers, Copenhagen, Denmark) with plenty of irrigation with water. The surfaces were polished by means of the use of abrasive SiC discs from 800 to 4000 grits (grain size) followed by final polishing steps carried out with diamond pastes from 1 µm to 0.25 µm (Struers LaboPol-4; Struers GmbH, Hanover, Germany).

All the samples were observed by means of AFM to evaluate the existence of tubular occlusion and those with occluded tubules were excluded from study.

The dentin samples were treated with a solution of citric acid (pH 3.8) for 1 minute to ensure the opening of the dentin tubules and eliminate the dentin smear layer. The dentin surfaces were rinsed before applying the samples/suspensions. Suspensions in PBS of unfunctionalized polymeric particles, polymeric particles functionalized with Ca⁺² and Zn⁺² at a concentration of 10 mg/mL or only one solution of buffer for the test of the untreated dentin were applied for 30 seconds.

The treated teeth were stored in phosphate buffer at 37°C for 24 hours and 7 days.

For the virtually static nanoindentation with the Berkovich diamond indentor, 10 indentations were carried out with a peak load of 4000 µN and an operating time of 10 s in each dentin sample in the PD and ID areas at 24 hours and 7 days of storage. Based on this test, the nanohardness (Hi) and Young's elasticity modulus (Ei) values of the samples were obtained. The indentor progressively pressed (at constant speed) the sample up to a maximum load of 4000 µN (load value of the experiment) and then the load was progressively released to the value zero (unloading value of the experiment). Based on the slope of these curves of Load vs. Depth, the nanoindentation values were obtained by means of applying the Oliver-Pharr method, which is based on a continuous, isotropic and homogeneous elastic contact model. The data were evaluated by means of ANOVA and Student-Newman-Keuls, presetting a value p < 0.05 to achieve statistical significance.

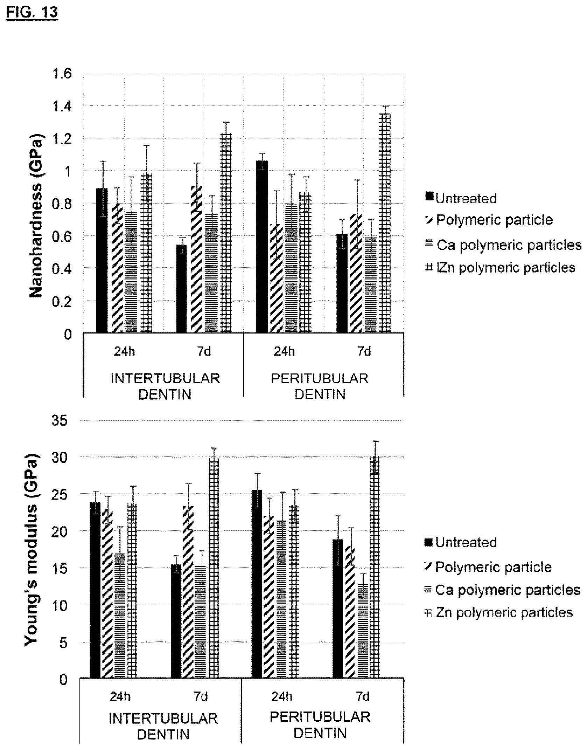

The results of this study showed that the nanohardness (Hi) and the Young's elasticity modulus (Ei) of the surface of the cervical dentin, obtained after applying polymeric spherical particles functionalized with Zn⁺², significantly increased after storage for 7 days, in comparison with samples treated for 24 hours. In addition, the dentin treated with polymeric particles functionalized with Zn⁺² achieved the highest Hi and Ei values of all the samples studied, after 7 days of storage (Fig. 13).

The increase in nanohardness (Hi) and Young's elasticity modulus (Ei) on the surface of the dentin is due to a remineralizing effect related to the appearance of mineral precipitates, fundamentally Ca, P and Zn, inside the demineralized organic matrix, in the intrafibrilar compartment. In fact, the extrafibrilar minerals act like a granular material which can support the load, but the intrafibrilar mineralization is the key factor to ensure that the collagen fibrils have the same high elasticity modulus and hardness as in the biomineralized natural dentin. The functional remineralization is the result of a process which causes the recovery of the lost physical and chemical properties in the dentin due to wear and erosion caused by foods and mastication. The most important point is that with the polymeric particles functionalized with Zn⁺² not only are the dentin tubules occluded, eliminating pain, but the increase in the mechanical properties of the dentin which generates zinc, will very likely cause the cessation or at least slowing of the dentin wear, stopping the progress of this disease. The polymeric particles functionalized with Zn⁺² are also inhibitory of the metalloproteases which are clearly involved in the rapid advance of this pathology, due to causing chemical degradation and hydrolysis of the eroded dentin. There is therefore a triple beneficial effect.

## Claims

1. A method for obtaining functionalized polymeric particles
• comprising a copolymer with statistical topology and a chemical structure (acid monomer selected from acrylic or methacrylic)-co-(cross-linking agent selected from acrylic or methacrylic)-co-(hydroxylated monomer selected from acrylic or methacrylic)
• having particle dimensions of less than 1000 nm
• being functionalized with a divalent cation selected from Zn⁺², Ca⁺², Mg⁺² and Sr⁺², an antibacterial agent and/or any of the combinations thereof,
**characterized by** being a precipitation polymerization comprising the following steps
a) preparing a solution comprising:
• an acid monomer selected from acrylic or methacrylic in a proportion of 0.05% and 10% by weight with respect to the total weight of the solution
• a cross-linking agent selected from acrylic or methacrylic in a proportion of 0.05% and 10% by weight with respect to the total weight of the solution
• a hydroxylated monomer selected from acrylic or methacrylic in a proportion of 0.05% and 10% by weight with respect to the total weight of the solution
• a solvent comprising at least acetonitrile, acrylonitrile, propionitrile, benzonitrile, butyronitrile, methyl ethyl ketone (butanone), ethyl acetate, 1,2-dimethoxyethane or a combination thereof, in a proportion between 80% and 99.8% by weight with respect to the total weight of the solution
• an initiator in a proportion of 0.01 % and 2% by weight with respect to the total weight of the solution
• particles with fractal surface in a proportion of 0.009% and 2% by weight with respect to the total weight of the solution
b) eliminating the oxygen present in the solution obtained in step (a);
c) heating the solution obtained in (b) to a temperature between 30°C and the boiling point of the solvent used in step (a);
d) isolating the polymeric particles obtained in step (c), rinsing and drying;
e) functionalizing the polymeric particles obtained in step (d) by means of incubating them in an aqueous solution comprising Zn⁺², Ca⁺², Mg⁺², Sr⁺² and/or an antibacterial agent to obtain a suspension, wherein the percentage by weight of the polymeric particles in the suspension is between 0.01% and 30%; and
f) isolating the polymeric particles functionalized in step (e) and drying.

2. The method according to claim 1, wherein the polymeric particles have particle dimensions of less than 300 nm.

3. The method according to any of claims 1 or 2, wherein the polymeric particles are spherical.

4. The method according to any of claims 1 to 3, wherein the acid monomer of step (a) is selected from methacrylic acid (MA), acrylic acid (AA), 2-carboxyethyl acrylate, mono-2-(methacryloyloxy)ethyl maleate, 2-carboxyethyl acrylate oligomers, 2-bromoacrylic acid, 2-bromomethacrylic acid, 2-ethylacrylic acid, 2-propylacrylic acid and 2-trifluoromethyl acrylic acid.

5. The method according to any of claims 1 to 4, wherein the cross-linking agent of step (a) is selected from ethylene glycol dimethacrylate (EDMA), 3-(acryloyloxy)-2-hydroxypropyl methacrylate, bis[2-(methacryloyloxy)ethyl] phosphate, bisphenol A propoxylate diacrylate, 1,3-butanediol diacrylate, 1,4 butanediol diacrylate, 1,3-butanediol dimethacrylate, di(trimethylpropane) tetracrylate, diurethane dimethacrylate, glycerol 1,3-diglycerolate diacrylate, glycerol dimethacrylate, glycerol propoxylate (1PO/OH) triacrylate, 1,6-hexanediol diacrylate, neopentyl glycol diacrylate, pentaerythritol diacrylate monostearate, pentaerythritol tetra-acrylate and trimethylolpropane propoxylate triacrylate.

6. The method according to any of claims 1 to 5, wherein the hydroxylated monomer of step (a) is selected from 2-hydroxyethyl methacrylate (HEMA), 2-hydroxyethyl acrylate (HEA), hydroxypropyl acrylate, hydroxypropyl methacrylate, 4-hydroxybutyl acrylate, hydroxybutyl methacrylate and 2-hydroxy-3-phenoxypropyl acrylate.

7. The method according to any of claims 1 to 6, wherein the solvent of step (a) is pure and is selected from acetonitrile, acrylonitrile, propionitrile, benzonitrile, butyronitrile, methyl ethyl ketone (butanone), ethyl acetate, 1,2-dimethoxyethane and a combination thereof.

8. The method according to any of claims 1 to 7, wherein the initiator of step (a) is selected from 2,2'-azobis(2-methylpropionnitrile) (AIBN), 1,1'azobis(cyclohexanecarbonitrile) (ACHN), 2,2'-azobis (2-methylpropionamidine) 2,2'-dihydrochloride (AAPH), 4,4'-azobis(4-cyanovaleric acid) (ACVA), tert-butyl hydroperoxide, cumene hydroperoxide, 2,5-di(tert-butylperoxide)-2,5-dimethyl-3-hexyne, dicumyl peroxide and 2,5-bis(tert-butylperoxide)-2,5-dimethylhexane.

9. The method according to any of claims 1 to 8, wherein the particles with fractal surface of step (a) are SiO₂ or Al₂O₃ nuclei with a particle size of between 1 nm and 400 nm.

10. The method according to claim 9, wherein the particle size of the SiO₂ or Al₂O₃ nuclei is between 1 nm and 50 nm.

11. The method according to any of claims 1 to 10, wherein the antibacterial agent of step (e) is selected from the list comprising tetracycline, oxytetracycline, doxycycline hyclate, doxycycline hydrochloride, 4-epi-chlortetracycline hydrochloride, neomycin, gentamycin, tobramycin, macrolides, penicillins, vancomycin, cephalosporins and non-toxic antiseptics for oral use chlorhexidine digluconate or chlorhexidine acetate.

12. Polymer particles obtained by means of the method described according to any of claims 1 to 11, wherein said particles
• comprise a copolymer with statistical topology and a chemical structure (acid monomer selected from acrylic or methacrylic)-co-(cross-linking agent selected from acrylic or methacrylic)-co-(hydroxylated monomer selected from acrylic or methacrylic)
• have particle dimensions of less than 1000 nm
• are functionalized with a divalent cation selected from Zn⁺², Ca⁺², Mg⁺² and Sr⁺², an antibacterial agent and/or any of the combinations thereof.

13. The particles according to claim 12, wherein the particle dimensions are less than 300 nm.

14. A composition comprising the particles according to any of claims 12 or 13.

15. A pharmaceutically-acceptable composition according to any of claims 12 or 13.

16. A use of the particles according to claim 12 for the manufacture of a medicament.

17. The use according to claim 16, for the manufacture of a medicament for the remineralization of the dentin, wherein the particles are functionalized with at least one divalent cation selected from Zn⁺², Ca⁺², Mg⁺² and Sr⁺².

18. The use according to claim 16, for the manufacture of a medicament for the treatment and/or prevention of bacterial infections of the root dentin, wherein the particles are functionalized with at least one antibacterial agent.

19. The use according to claim 16, for the manufacture of a medicament for the remineralization of the dentin and for the treatment and/or prevention of bacterial infections of the root dentin, wherein the particles are functionalized with at least one divalent cation selected from Zn⁺², Ca⁺², Mg⁺² and Sr⁺² and with at least one antibacterial agent.

20. The use according to claim 16, for the manufacture of a medicament for the treatment and/or prevention of the hypersensitivity of the dentin.

21. The use according to claim 16, for the manufacture of a medicament for the treatment and/or prevention of infectious recurrences following an endodontic intervention.
